# EUROPEAN PATENT APPLICATION

(11) **EP 4 810 069 A1**
(43) Date of publication of application: **23.09.2026**
(21) Application number: 25165022.2
(22) Date of filing: 20.03.2025
(51) Int. Cl.: A61K 51/10, A61K 101/02, A61K 103/00

(54) **IMAGING AGENTS FOR GPC3**

(71) Applicant: Bayer Aktiengesellschaft, 51373 Leverkusen (DE); Bayer AS, 0283 Oslo (NO)
(72) Inventor: Mahlert, Christoph, 51373 Leverkusen (DE); Trautwein, Mark, 51373 Leverkusen (DE); Michalski, Sebastian, 51373 Leverkusen (DE); Karlsson, Jenny, 0283 Oslo (NO); Biseth, Katrine Wickstroem, 0283 Oslo (NO); Suurs, Frans Valentijn, 0283 Oslo (NO)
(74) Representative: BIP Patents

(57) **Abstract**

The present disclosure relates to an imaging agent comprising a targeting moiety capable of binding GPC3, a chelator and a radionuclide.

## Description

### FIELD OF THE INVENTION

The disclosure relates to novel imagining agents for GPC3 preferably PET imaging agents.

### BACKGROUND

Glypican-3 (GPC3) is an oncofetal protein expressed in various tumors, for example hepatocellular carcinoma (HCC), non-small cell lung cancer (NSCLC), gastric carcinoma, ovarian carcinoma, melanomas, and pediatric embryonal tumors. GPC3 expression is particularly high in HCC with limited expression in adults and it is not expressed in viral hepatitis, cirrhosis or NASH, making HCC a highly attractive target for GPC3 based therapy.

Several radiolabeled probes targeting GPC3 have been reported for imaging of GPC3 positive tumors. WO2014159087 for example disclosed a radiolabeled anti-glypican-3 immunoconjugate for immuno-PET imaging of hepatocellular carcinoma in particular a construct consisting of ⁸⁹Zr-DFO-GPC3 Ab. Moreover, the document CN116514984 A disclosed an ¹⁸ F-labeled GPC3 nanobody i.e. VHH probe with a DBCO chelator and also An et al. 2022 developed GPC3-VHH-immune-PET probes. Li et al 2020 as well as Mo et al. 2024, on the other hand, reported radiofluorinated GPC3-binding peptides for PET Imaging of hepatocellular carcinoma. In addition, CN117247454 A mentioned a ⁶⁸Ga-NOTA-aGPC3-Fab and a ⁶⁸ Ga-aGPC3-scFv.

Despite these existing radiolabeled probes targeting GPC3, a clinical need remains to find an imaging method that can perform HCC diagnosis early and accurately in order to assist in treatment planning and maximizing patient prognosis while demonstrating a good patient safety profile.

### SUMMARY OF THE INVENTION

What is described herein relates in a first aspect to an imaging agent comprising a targeting moiety capable of binding GPC3, a chelator and a radionuclide, as well as a method for producing said imaging agent (second aspect), a kit comprising said imagining agent (third aspect) and uses of said imaging agent (fourth aspect).

### DEFINITIONS

Unless defined otherwise, all technical and scientific terms used herein have the meaning commonly understood by one of ordinary skill in the art to which this description belongs. As used herein and in the appended claims, the singular forms "a," and "the" include plural reference unless the context clearly dictates otherwise. Thus, for example, reference to "a gene" is a reference to one or more genes and includes equivalents thereof known to those skilled in the art, and so forth. In the context of the present disclosure, the term "comprises" or "comprising" means "including, but not limited to". The term is intended to be open-ended, to specify the presence of any stated features, elements, integers, steps or components, but not to preclude the presence or addition of one or more other features, elements, integers, steps, components or groups thereof. The term "comprising" thus includes the more restrictive terms "consisting of" and "essentially consisting of". In one embodiment the term "comprising" as used throughout the application and in particular within the claims may be replaced by the term "consisting of".

In this context, the term "about" or "approximately" means within 80% to 120%, alternatively within 90% to 110%, including within 95% to 105% of a given value or range. If within the present text any item is referred to as "as mentioned herein", it means that it may be mentioned anywhere in the present text.

### Target

In the context of the disclosure, the term "GPC3" refers to the glypican 3. GPC3 is an oncofetal heparan sulfate glycoprotein attached to the cell membrane by a glycophosphatidylinositol anchor (Filmus J, Capurro M. Glypican-3: a marker and a therapeutic target in hepatocellular carcinoma. FEBS J. 2013 May;280(10):2471-6). GPC3 contains 580 amino acids and has a molecular weight of 70 kDA. Two HS chains are attached near the C-terminal portion. The single-chain GPC3 is processed by furin at the Arg358-Cys359 bond to generate the mature GPC3 consisting of a 40-kDa N-terminal subunit and a 30-kDa C-terminal subunit linked by disulfide bonds (De Cat B, Muyldermans SY, Coomans C, Degeest G, Vanderschueren B, Creemers J, Biemar F, Peers B, David G. Processing by proprotein convertases is required for glypican-3 modulation of cell survival, Wnt signaling, and gastrulation movements. J Cell Biol. 2003 Nov 10;163(3):625-35). Specific binding fragments such as Fab, Fab', F(ab')2 and single-chain specific binding antibodies are typical fragments. The human GPC3 protein is encoded by the gene GPC3 (NCBI Gene ID 2719) (SEQ ID NO: 42). Synonyms for GPC3 are SGB, DGSX, MXR7, SDYS, SGBS, OCI-5, SGBS1 and GTR2-2. The GPC3 protein comprises human, murine, cynomolgus and further mammalian and non-mammalian homologues.

### Biological subject-matter

The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers. Unless otherwise indicated, a particular polypeptide sequence also implicitly encompasses conservatively modified variants thereof.

Amino acids may be referred to herein by their commonly known three letter symbols or by the one-letter symbols recommended by the IUPAC-IUB Biochemical Nomenclature Commission. Nucleotides, likewise, may be referred to by their commonly accepted single-letter codes.

The term "mutein" or "variant" can be used interchangeably and refers to an antibody or antigen-binding fragment that contains at least one amino acid substitution, deletion, or insertion in the variable region or the portion equivalent to the variable region, provided that the mutein or variant retains the desired binding affinity or biological activity. Variants of the antibodies or antigen-binding antibody fragments contemplated in the invention are molecules in which the binding activity of the antibody or antigen-binding antibody fragment is maintained.

An "isolated" nucleic acid is one that has been identified and separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "germlining" refers to replacement of residues in the variable domains of an antibody with those present in the pre-mutated germline genes to reduce the potential for immunogenicity.

"Percent (%) sequence identity" with respect to a reference polynucleotide or polypeptide sequence, respectively, is defined as the percentage of nucleic acid or amino acid residues, respectively, in a candidate sequence that are identical with the nucleic acid or amino acid residues, respectively, in the reference polynucleotide or polypeptide sequence, respectively, after aligning the sequences and introducing gaps, if necessary, to achieve the maximum percent sequence identity. Conservative substitutions are not considered as part of the sequence identity. Preferred are un-gapped alignments. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full-length of the sequences being compared.

Substantial sequence identity/similarity may be taken as having a sequence similarity/identity of at least 80% to the complete sequences and/or at least 90% to the specific binding regions (e.g., the CDR regions). Preferable sequence similarity or more preferably identity may be at least 92%, 95%, 97%, 98% or 99%. Sequence similarity and/or identity may be determined using the "BestFit" program of the Genetics Computer Group Version 10 software package from the University of Wisconsin. The program uses the local had algorithm of Smith and Waterman with default values: Gap creation penalty=8, Gap extension penalty=2, Average match=2.912, average mismatch 2.003.

The terms "polynucleotide" or "nucleic acid", as used interchangeably herein, refer to chains of nucleotides of any length, and include DNA and RNA. The nucleotides can be deoxyribonucleotides, ribonucleotides, modified nucleotides or bases, and/or their analogs, or any substrate that can be incorporated into a chain by DNA or RNA polymerase. A polynucleotide may comprise modified nucleotides, such as methylated nucleotides and their analogs.

"Sequence homology" indicates the percentage of amino acids that either is identical or that represent conservative amino acid substitutions.

The term "vector", as used herein, refers to a nucleic acid molecule capable of propagating another nucleic acid to which it is linked. The term includes the vector as a self- replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The terms "host cell", "host cell line", and "host cell culture" are used interchangeably and refer to cells into which at least one exogenous nucleic acid has been introduced, including the progeny of such cells. Host cells include "transformants" and "transformed cells", "transfectants" and "transfected cells" and "transduced cells" which include the primary transformed/transfected/transduced cell and progeny derived therefrom without regard to the number of passages. Progeny may not be completely identical in nucleic acid content to a parent cell but may contain mutations. Mutant progeny that have the same function or biological activity as screened or selected for in the originally transformed cell are included herein.

### Antibodies

The term "antibody", as used herein, is intended to refer to immunoglobulin molecules including, but not limited to, full-length antibodies and monovalent antibodies. "Full-length antibodies" are preferably comprised of four polypeptide chains, two heavy (H) chains and two light (L) chains which are typically inter-connected by disulfide bonds. Each heavy chain is comprised of a heavy chain variable region (abbreviated herein as VH) and a heavy chain constant region. The heavy chain constant region can comprise e.g., three domains CH1, CH2 and CH3. Each light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs arranged from amino-terminus to carboxy-terminus e.g., in the following order: FR1, CDR1, FR2, CDR2, FR3, CDR3, FR4.

"Monovalent antibodies" as used herein are preferably comprised of three polypeptide chains, two heavy (H) chains and one light (L) chain which are typically inter-connected by disulfide bonds. One heavy chain is comprised of a heavy chain variable region (abbreviated herein as VH) and a heavy chain constant region. The heavy chain constant region can comprise e.g., three domains CH1, CH2 and CH3. The other heavy chain is comprised of a heavy chain constant region only. The light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs arranged from amino-terminus to carboxy-terminus e.g., in the following order: FR1, CDR1, FR2, CDR2, FR3, CDR3, FR4.

As used herein, the term "Complementarity Determining Regions" (CDRs; e.g., CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat (e.g. about residues 24-34 (L1), 50-56 (L2) and 89-97 (L3) in the light chain variable domain and 31-35 (H1), 50-65 (H2) and 95-102 (H3) in the heavy chain variable domain; (Kabat et al., Sequences of Proteins of Immunolological Interest, 5th Ed. Public Health Service, National Institutes of Health, Bethesda, MD. (1991)) and/or those residues from a "hypervariable loop" (e.g. about residues 26-32 (L1), 50-52 (L2) and 91-96 (L3) in the light chain variable domain and 26-32 (H1), 53-55 (H2) and 96-101 (H3) in the heavy chain variable domain (Chothia and Lesk; J Mol Biol 196: 901-917 (1987)). In some instances, a complementarity determining region can include amino acids from both a CDR region defined according to Kabat and a hypervariable loop.

Depending on the amino acid sequence of the constant domain of their heavy chains, intact antibodies can be assigned to different "classes". There are five major classes of intact antibodies: IgA, IgD, IgE, IgG, and IgM, and several of these maybe further divided into "subclasses" (isotypes), e.g., IgG1, IgG2, IgG3, IgG4, IgA1, and IgA2. A preferred class of immunoglobulins for use of what is described herein is IgG. In particular embodiments, the antibody according to the present disclosure is an IgG1, an IgG2, an IgG3 or an IgG4 antibody, more particularly an IgG1 antibody. Different isotypes may have different effector functions. Human light chains are classified as kappa (K) and lambda (λ) light chains. Within light and heavy chains, the variable and constant regions are joined by a "J" region of about 12 or more amino acids, with the heavy chain also including a "D" region of about 10 more amino acids. See generally, Fundamental Immunology, Ch. 7 (Paul, W., ed., 2nd ed. Raven Press, N.Y. (1989)). The heavy-chain constant domains that correspond to the different classes of antibodies are called [alpha], [delta], [epsilon], [gamma], and [mu], respectively. The subunit structures and three-dimensional configurations of different classes of immunoglobulins are well known. As used herein antibodies are conventionally known antibodies and functional fragments thereof.

The terms "anti-GPC3 antibody" or "anti-glypican 3 antibody" and "an antibody that binds to glypican 3" or "an antibody that binds to GPC3" are used synonymously herein and refer to an antibody that is capable of binding GPC3, preferably with sufficient affinity such that the antibody is useful as a diagnostic and/or therapeutic agent in targeting GPC3.

"Codrituzumab" (CAS Registry Number 1365267-33-9), is a humanized monoclonal anti-GPC3 antibody. Its preparation, isolation and purification has been described in e.g., US7,919,086. Codrituzumab (also named "TPP-14890" throughout the present description) comprises a heavy chain protein sequence according to SEQ ID NO. 9 and a light chain protein sequence according to SEQ ID NO.10.

As used herein, an antibody "binds specifically to", is "specific to/for" or "specifically recognizes" an antigen of interest, e.g. GPC3, is one that binds the antigen-target with sufficient affinity such that the antibody is useful as a diagnostic or therapeutic agent in targeting a cell or tissue expressing the antigen or one that binds an antigen-binding polypeptide target with sufficient affinity such that the antibody is useful as a reversal agent to neutralize the therapeutic activity of this antigen-binding polypeptide (e.g. an antigen-binding antibody) and does not significantly cross-react with other proteins or does not significantly cross-react with proteins other than orthologs and variants (e.g. mutant forms, splice variants, or proteolytically truncated forms) of the aforementioned target. The term "specifically recognizes" or "binds specifically to" or is "specific to/for" a particular polypeptide or an epitope on a particular polypeptide target as used herein can be exhibited, for example, by an antibody, or antigen-binding fragment thereof, having a monovalent dissociation constant (K_{D}) for the antigen of less than about 10⁻⁴ M, alternatively less than about 10⁻⁵ M, alternatively less than about 10⁻⁶ M, alternatively less than about 10⁻⁷ M, alternatively less than about 10⁻⁸ M, alternatively less than about 10⁻⁹ M, alternatively less than about 10⁻¹⁰ M, alternatively less than about 10⁻¹¹ M, alternatively less than about 10⁻¹² M, or less. An antibody "binds specifically to," is "specific to/for" or "specifically recognizes" an antigen if such antibody is able to discriminate between such antigen and one or more reference antigen(s). In its most general form, "specific binding", "binds specifically to", is "specific to/for" or "specifically recognizes" is referring to the ability of the antibody to discriminate between the antigen of interest and an unrelated antigen, as determined, for example, in accordance with one of the following methods. Such methods comprise, but are not limited to, surface plasmon resonance (SPR), Western blots, ELISA-, RIA-, ECL-, IRMA-tests and peptide scans. For example, a standard ELISA assay can be carried out. The scoring may be carried out by standard color development (e.g., secondary antibody with horseradish peroxidase and tetramethyl benzidine with hydrogen peroxide). The reaction in certain wells is scored by the optical density, for example, at 450 nm. Typical background (=negative reaction) may be 0.1 OD; typical positive reaction may be 1 OD. This means the difference positive/negative is more than 5-fold, 10-fold, 50-fold, and preferably more than 100-fold. Typically, determination of binding specificity is performed by using not a single reference antigen, but a set of about three to five unrelated antigens, such as milk powder, BSA, transferrin or the like.

### Antibody fragments

A "functional fragment" or "antigen-binding antibody fragment" of an antibody/immunoglobulin hereby is defined as a fragment of an antibody/immunoglobulin (e.g., a variable region of an IgG) that retains the antigen-binding region. An "antigen-binding region" of an antibody typically is found in one or more hyper variable region(s) of an antibody, e.g., the CDR1, -2, and/or -3 regions; however, the variable "framework" regions can also play an important role in antigen binding, such as by providing a scaffold for the CDRs. Preferably, the "antigen-binding region" comprises at least amino acid residues 4 to 103 of the variable light (VL) chain and 5 to 109 of the variable heavy (VH) chain, more preferably amino acid residues 3 to 107 of VL and 4 to 111 of VH, and particularly preferred are the complete VL and VH chains (amino acid positions 1 to 109 of VL and 1 to 113 of VH; numbering according to WO 97/08320).

Nonlimiting examples of "functional fragments" or "antigen-binding antibody fragments" include Fab, Fab', F(ab')2, Fv fragments, domain antibodies (dAb), complementarity determining region (CDR) fragments, single-chain antibodies (scFv), single chain antibody fragments, diabodies, triabodies, tetrabodies, minibodies, linear antibodies (Zapata et al., Protein Eng.,8 (10): 1057-1062 (1995)); chelating recombinant antibodies, tribodies or bibodies, intrabodies, nanobodies, small modular immunopharmaceuticals (SMIPs), an antigen-binding-domain immunoglobulin fusion protein, a camelized antibody, a VHH containing antibody, or muteins or derivatives thereof, and polypeptides that contain at least a portion of an immunoglobulin that is sufficient to confer specific antigen binding to the polypeptide, such as a CDR sequence, as long as the antibody retains the desired biological activity; and multispecific antibodies such as bi- and tri-specific antibodies formed from antibody fragments (C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag).

An antibody other than a "bispecific" or "bifunctional" antibody is understood to have each of its binding sites identical.

The F(ab')₂ or Fab may be engineered to minimize or completely remove the intermolecular disulfide interactions that occur between the C_{H1} and C_{L} domains. Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two "Fv" fragments. An "Fv" fragment is the minimum antibody fragment that contains a complete antigen recognition and binding site. This region consists of a dimer of one heavy- and one light-chain variable domain in tight, non-covalent association. It is in this configuration that the three CDRs of each variable domain interact to define an antigen binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen.

"Single-chain Fv" or "sFv" or "scFv" antibody fragments comprise the VH and VL domains of antibody, wherein these domains are present in a single polypeptide chain.

Preferably, the Fv polypeptide further comprises a polypeptide linker between the VH and VL domains that enables the Fv to form the desired structure for antigen binding. For a review of Fvs see Pluckthun in The Pharmacology of Monoclonal Antibodies, vol. 113, Rosenburg and Moore eds., Springer-Verlag, New York, pp. 269-315 (1994).

The Fab fragment also contains the constant domain of the light chain and the first constant domain (CH1) of the heavy chain. Fab fragments differ from Fab' fragments by the addition of a few residues at the carboxyl terminus of the heavy chain CH1 domain including one or more cysteine residues from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteine residues between them.

A "targeting moiety" as used herein is any chemical structure binding to a biological target, such as GPC3 or a cell expressing GPC3. The targeting moiety localizes itself, e.g. as part of a bigger structure at a specific site where the presence is required to exert the intended effect, e.g. delivery of radioactive decay in case of a TAC or an imaging agent in PET/CT. Thus, a tissue targeting group or moiety serves to provide greater localization of a molecule or conjugate to at least one desired site in the body of a subject in comparison with the concentration of an equivalent compound not comprising the targeting moiety. The targeting moiety can be for example selected without limitation from the group consisting of nucleotides, DNA and RNA fragments, aptamers, peptides, proteins, antibodies or fragments thereof especially a Fab, nanoparticles, or small molecules, or any combination thereof. According to what is described herein, the targeting moiety is preferably a Fab.

### Binding

"Binding affinity" or "affinity" refers to the strength of the total sum of non-covalent interactions between a single binding site of a molecule and its binding partner. Unless indicated otherwise, as used herein, "binding affinity" refers to intrinsic binding affinity which reflects a 1:1 interaction between members of a binding pair (e.g., an antibody and an antigen). The affinity of an antibody or fragment thereof for a target can be determined using techniques well known in the art, for example by ELISA, isothermal titration calorimetry (ITC), surface plasmon resonance (SPR), flow cytometry or fluorescent polarization assays. The dissociation constant "K_{D}" is commonly used to describe the affinity between a molecule (such as an antibody) and its binding partner (such as an antigen) i.e., how tightly a ligand binds to a particular protein. Ligand-protein affinities are influenced by non-covalent intermolecular interactions between the two molecules. Affinity can be measured by common methods known in the art, including those described herein. Preferably the affinity is provided as dissociation constant K_{D}, in the alternative the affinity is provided as an EC50 value. In one embodiment, the "K_{D}" or "K_{D} value" according to this invention is measured by using surface plasmon resonance assays a Biacore T200 instrument (Cytiva). Other suitable devices are Biacore X100, Biacore S200, Biacore 8K, Biacore 8K+, Biacore 4000 SPR (Cytiva), Alto Digital SPR (Nicoya Lifesciences Inc.), or SIERRA SPR-32 PRO (Bruker Corporation).

"Half maximal effective concentration" (EC50) refers to the concentration of a drug, modulator, antibody, fragment, conjugate or molecule which induces a response halfway between the baseline and maximum after a specified incubation time. In the context of affinity, the EC50 thus reflects the concentration of binder (e.g. antibody) that is needed for half-maximal binding. An EC50 can be determined if an inflection point can be determined by mathematical modeling (e.g., non-linear regression) of the dose-response curve describing the relationship between applied drug, antibody, fragment, conjugate or molecule concentration and signal. For example, if the dose-response curve follows a sigmoidal curve, an EC50 can be determined. Where the response is an inhibition, the EC50 is termed "half maximal inhibitory concentration" (IC50).

As used herein, the term "epitope" includes any protein determinant capable of specific binding to an immunoglobulin or T-cell receptor. Epitopic determinants usually consist of chemically active surface groupings of molecules such as amino acids or sugar side chains, or combinations thereof and usually have specific three-dimensional structural characteristics, as well as specific charge characteristics.

### Antibody formats

A "humanized antibody" is generally defined as one that is (I) derived from a non-human source (*e*.*g*., a transgenic mouse which bears a heterologous immune system), which antibody is based on a human germline sequence; or (II) CDR-grafted, wherein the CDRs of the variable region are from a non-human origin, while one or more framework regions and/or part of the CDR sequence of the variable region are of human origin and typically the constant region (if any) is of human origin.

The term "humanized antibody" thus includes antibodies in which the variable region in either the heavy or light chain or both of a human antibody is altered by at least partial replacement of one or more CDRs from a non-human antibody of known specificity and, if necessary, by partial framework region replacement and sequence changing. In other words, an antibody in which one or more "donor" CDRs from a non-human antibody (such as mouse, rat, rabbit or non-human primate antibody) of known specificity is grafted into a human heavy or light chain framework region is referred to herein as a "humanized antibody." It may not be necessary to replace all of the CDRs with the complete CDRs from the donor variable domain to transfer the antigen binding capacity of one variable domain to another. Rather, it may only be necessary to transfer those residues that are necessary to maintain the activity of the target binding site.

The framework regions (FR) within the variable region in a heavy or light chain, or both, of a humanized antibody may comprise solely residues of human origin, in which case these framework regions of the humanized antibody are referred to as "fully human framework regions." A human framework region that comprises a mixture of human and donor framework residues,and is referred to herein as a "partially human framework region." Furthermore, humanized antibodies may comprise residues that are neither found in the recipient antibody nor in the donor antibody. These modifications are made to further refine antibody performance (e.g., to obtain desired affinity).

In general, the humanized antibody will thus comprise at least one, and typically two, variable regions, in which all or part of the CDRs correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details see Jones et al., Nature 331:522-25 (1986); Riechmann et al., Nature 332:323-27 (1988); and Presta, Curr. Opin. Struct. Biol. 2:593-96 (1992), each incorporated herein by reference.

"Human antibodies" or "fully human antibodies" comprise human derived CDRs, i.e. CDRs of human origin. A "human" antibody is hereby defined as one that is not chimeric or "humanized" and not from (either in whole or in part) a non-human species. A human antibody or functional antibody fragment can be derived from a human or can be a synthetic human antibody. A "synthetic human antibody" is defined herein as an antibody having a sequence derived, in whole or in part, *in silico* from synthetic sequences that are based on the analysis of known human antibody sequences. *In silico* design of a human antibody sequence or fragment thereof can be achieved, for example, by analyzing a database of human antibody or antibody fragment sequences and devising an amino acid sequence utilizing the data obtained therefrom. Another example of a human antibody or functional antibody fragment is one that is encoded by a nucleic acid isolated from a library of antibody sequences of human origin (*i.e.,* such library being based on antibodies taken from a human natural source).

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the term "monoclonal" indicates the character of the antibody as not being a mixture of discrete antibodies. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins. The term "monoclonal" is not to be construed as to require production of the antibody by any particular method. For example, the monoclonal antibodies to be used may be made by the hybridoma method first described by Kohler et al., Nature, 256: 495 [1975, or may be made by recombinant DNA methods (see, e.g., U.S. Patent No. 4,816,567). The "monoclonal antibodies" may also be recombinant, chimeric, humanized, human, Human Engineered^{™}, or antibody fragments, for example.

An "isolated" antibody is one that has been identified and separated from a component of the cell that expressed it. Contaminant components of the cell are materials that would interfere with diagnostic or therapeutic uses of the antibody, and may include enzymes, hormones, and other proteinaceous or non-proteinaceous solutes.

### Antibody conjugate

The term "antibody conjugate" refers to a conjugate comprising at least one antibody moiety and one or more further molecules or moieties. The one or more further molecules or moieties may be selected without limitation from a drug, a chelator, a radioactive element, a cytotoxic agent, a further antibody or antigen-binding fragment.

The term "antibody drug conjugate" ("ADC") refers to an antibody conjugate comprising at least one drug moiety.

A "targeted actinium conjugate" (also "TAC") is a conjugate comprising a targeting moiety (i.e., an Fab) and at least one chelator or chelating group for complexation of actinium (e.g. macropa, preferably Macropa-NCS), and optionally comprise actinium.

### PET Imaging

Positron emission tomography (PET) is a medical imaging technique that detects gamma photons produced by positron emitters and reconstructs these signals to visualize the location of the positron emitter. When combined with computed tomography (CT), PET/CT allows for the precise localization of the positron emitter within the anatomical framework of the body. Multiple positron emitters are routinely used in the clinic, such as C-11, F-18, Ga-68 and Zr-89.

### Radionuclide

A "radionuclide" (also: "radioactive nuclide", "radioisotope" or "radioactive isotope") is an atom that undergoes radioactive decay. Without limitation, for example the radionuclide may be a β-emitting radionuclide (beta emitter), an α-particle-emitting radionuclide (alpha emitter), or an Auger electron emitting radionuclide (Auger electron emitter).

"β-emitting radionuclides" or "beta emitter" are radionuclides which emit beta particles. Examples of beta emitters include without limitation copper-67 (⁶⁷Cu), strontium-89 (⁸⁹Sr), yttrium-90 (⁹⁰Y), rhodium-105 (¹⁰⁵Rh), iodine-131 (¹³¹I), promethium-149 (¹⁴⁹Pm), holmium-166 (¹⁶⁶Ho), lutetium-177 (¹⁷⁷Lu), rhenium-186 (¹⁸⁶Re), rhenium-188 (¹⁸⁸Re), gold-198 (¹⁹⁸Au) and gold-199 (¹⁹⁹Au). Jongho has reviewed various techniques for chelation of various of these radioisotypes (Jeon, Jongho. "Review of therapeutic applications of radiolabeled functional nanomaterials." International journal of molecular sciences 20.9 (2019): 2323.).

"Auger electron emitting radionuclides" or "Auger electron emitter" are radionuclides which emit Auger electrons. Examples of Auger electron emitters include without limitation bromine-77, indium-111, iodine-123, and iodine-125.

"α-emitting radionuclides" or "alpha emitter" are radionuclides which emit alpha particles, i.e. 4He nuclei with a +2 charge. Non limiting examples of alpha emitters include bismuth-213 (²¹³Bi), characterized by a half-life of 45.6 min, actinium-225 (²²⁵Ac), characterized by a half-life of 9.9 d, astatine-211 (²¹¹At), characterized by a half-life of 7.2 h, radium-223 (²²³Ra), characterized by a half-life of 11.4 d, radium-224 (²²⁴Ra), characterized by a half-life of 3.7 d, and thorium-227 (²²⁷Th), characterized by a half-life of 18.7 d.

In the context of what is described herein the term "F" refers to at least one beta emitting fluorine isotope also termed fluorine radionuclide. Preferably the beta emitting fluorine isotope is 18F also termed "¹⁸F" or "radiofluorine".

Radionuclides can be obtained as known in the art. For example fluorine-18 can be made using either a cyclotron or linear particle accelerator to bombard a target such as enriched [¹⁸O]water.

### Chelator

"Chelation" refers to the formation or presence of two or more separate coordinate bonds between a ligand and a single central metal atom. The ligands which are capable of forming these coordinate bonds are termed "chelators", "chelating agents", or "sequestering agents". A "chelator" is hence a moiety - a chelating moiety- which is capable of forming - i.e. chelating - two or more separate coordinate bonds with a given radionuclide or group of radionuclides, such as, without limitation, alpha emitters, beta emitters or Auger electron emitter. Various chelators are known in the art and can be used according to the current invention, e.g. as described in Price, Eric W., and Chris Orvig. "Matching chelators to radiometals for radiopharmaceuticals." Chemical Society Reviews 43.1 (2014): 260-290, incorporated herein in its entirety.

Herein the terms chelating and radiolabeling are used interchangeably and refer to the process of coordinate bond formation between one or more radionuclides such as ¹⁸F optionally existing as a complex e.g. in the case of ¹⁸F with aluminum (Al) as [18F]AIF, to one or more chelator-antigen-targeting moieties. Such processes are known in the state of the art.

DFO* is an extended version of DFO and ⁸⁹Zr-DFO* compounds are shown to exhibit increased *in vitro* stability and improved *in vivo* properties compared to DFO compounds (WO2021051168) and therefore is a preferred chelator for Zr-89 when conjugated with antibodies or antibody fragments according to the present disclosure for use in PET imaging. DFO* refers to 5,11,16,22-Tetraazahexacosanediamide, N¹-[5-(acetylhydroxyamino)pentyl]-N²⁶,5,16-trihydroxy-N²⁶-[5-[[[(4-isothiocyanatophenyl)amino]thioxomethyl]amino]pentyl]-4,12,15,23-tetraoxo (CAS no.1810009-29-0).

As used herein the term "Resca" or "RESCA" refers to (±)-H3RESCA-TFPIs (N-{(1R,2R)-2-[bis(carboxymethyl)amino]cyclohexyl}-N-{4-[2-oxo-2-(2,3,5,6tetrafluorophenoxy)ethyl]benzyl} glycine (CAS# 1919794-40-3)

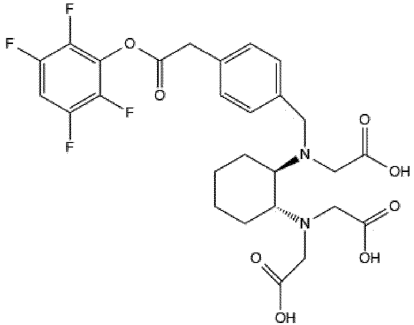

If ¹⁸F is used, a radiolabeling method as described in example 8 can be employed (cf. *also* Cleeren et al. Al18F-Labeling Of Heat-Sensitive Biomolecules for Positron Emission Tomography Imaging. Theranostics 2017; 7(11):2924-2939. doi:10.7150/thno.20094)*.* To a skilled person it is clear that if RESCA is used for radiolabelling of ¹⁸F according to this method then ¹⁸F is the radionuclide and RESCA the chelator with the Al still present within the construct as a whole. Thus, terms such as ¹⁸F -RESCA-anti-GPC3-Fab are understood to also still include the Al in case a radiolabelling method base on the Al18F-RESCA method was employed.

In the context of the present disclosure, the term CAR refers to the Chelator-to- antigen-targeting moiety ratio which quantifies the number of chelators attached to an antigen-targeting moiety molecule e.g. a Fab fragment.

In the context of what is described herein, the term IRF refers to the Immunoreactive Fraction i.e., the fraction of the radiolabeled targeting moieties which is capable of binding to the target (i.e., GPC3). The Lindmo assay (Lindmo T., et al. (1984) "Determination of the immunoreactive fraction of radiolabeled monoclonal antibodies by linear extrapolation to binding at infinite antigen excess." J. Immunol. Methods. 72, 77-89) is the most commonly used method for assessing the immunoreactive fraction.

### Imaging agent

As used herein the term "imaging agent" refers to a compound comprising a targeting moiety such as a Fab and radionuclide which emits radiation itself, which is different from contrast media which absorb or alter external electromagnetism or ultrasound. For example the imaging agent consists of a targeting moiety conjugated to a chelator which chelates the radionuclide which emits radiation itself.

In case Zr-89 is used as radionuclide suitable chelators for Zr-89 in PET imaging applications include, but are not limited to, for example DFO (Desferrioxamine), N-succinyl-desferrioxamine-tetrafluorophenol ester (N-suc-DFO-TFP ester) p-isothiocyanatobenzyl-desferrioxamine (DFO-Bz-NCS, also known as DFO-Ph-NCS), desferrioxamine-maleimide (DFO-maleimide), desferrioxamine-squaramide (DFO-sq), or DFO*.

In case ¹⁸F is used as radionuclide suitable chelators for ¹⁸F in PET imaging applications include, but are not limited to, for example RESCA.

### EMBODIMENTS

### Imaging agent (First Aspect)

In a first aspect what is described herein relates to an imaging agent comprising a targeting moiety capable of binding GPC3, a chelator and a radionuclide.

In a preferred embodiment of the first aspect the radionuclide is selected from the group consisting of ¹⁸ F, ⁴³Sc, ⁴⁴Sc, ⁴⁷Sc, ⁶⁷Cu, ⁶⁷Ga, ⁷¹Ge, ⁷⁷Br, ⁸⁹Sr, ⁸⁹Zr, ⁹⁰Y, ^{99m}_{Tc}, ¹⁰³Pd, ¹⁰⁵Rh,¹¹¹In, ¹²³I, ¹²⁵I, ²²⁷Th, ¹³¹I, ¹⁴⁰Nd, ¹⁴⁹Tb, ¹⁴⁹Pm, ¹⁵²Tb, ¹⁵⁵Tb, ¹⁶¹Tb, ¹⁶⁶Ho, ¹⁷⁷Lu, ¹⁷⁸Ta, ¹⁸⁶Re, ¹⁸⁸Re, ¹⁹³Pt, ^{195m}Pt, ¹⁹⁷Hg, ¹⁹⁸Au, ²¹¹At, ²¹²Bi, ²¹²Pb, ²¹³Bi, ²²³Ra, ²²⁴Ra, ²²⁵Ac and ²³²Th.

In a further preferred embodiment of the first aspect the radionuclide is detectable by positron emission tomography (PET) or single-photon emission computerized tomography (SPECT).

In another preferred embodiment of the first aspect the radionuclide is ⁸⁹Zr or¹⁸ F. Both of these radionuclides have half-lifes suitable for imaging applications especially for PET imaging.

In yet a further preferred embodiment of the first aspect the chelator is selected from the group consisting of Macropa (N,N'-bis[(6-carboxy-2-pyridil)methyl]-4,13-diaza-18-crown-6), DOTA (2,2',2"2‴-1,4,7,10-tetraazacyclododecane-1,4,7,10-tetrayl)tetraaceticacid or 1,4,7,10-tetra-azacyclo-ododecane-N,N',N",N‴-tetraacetic acid), DFO (Desferrioxamine), N-succinyl-desferrioxamine-tetrafluorophenol ester (N-suc-DFO-TFP ester) p-isothiocyanatobenzyl-desferrioxamine (DFO-Bz-NCS, also known as DFO-Ph-NCS), desferrioxamine-maleimide (DFO-maleimide), desferrioxamine-squaramide (DFO-sq), or DFO* (5,11,16,22-Tetraazahexacosanediamide, N¹-[5-(acetylhydroxyamino)pentyl]-N²⁶,5,16-trihydroxy-N²⁶-[5-[[[(4-isothiocyanatophenyl)amino] thioxomethyl]amino]pentyl]-4,12,15,23-tetraoxo), Nota (1,4,7-Triazacyclononane-1,4,7-triacetic acid) and RESCA. All these chelators can be conjugated with targeting moieties such as antibodies or antibody fragments capable of binding GPC3 to provide a means of targeting the radionuclide to the tumor to be imaged. According to a preferred embodiment of the present disclosure the chelators are conjugated the to the rest of the molecule via a terminal amino group of a lysine residue of the targeting moiety capable of binding GPC3.

It is clear to a skilled person that the choice of chelator depends on the chosen radionuclide e.g. as described in Price, Eric W., and Chris Orvig. "Matching chelators to radiometals for radiopharmaceuticals." Chemical Society Reviews 43.1 (2014): 260-290, incorporated herein in its entirety.

In an especially preferred embodiment of the first aspect the radionuclides are ⁸⁹Zr or¹⁸ F and the chelator is RESCA in the case of ¹⁸F or DFO* in the case of ⁸⁹Zr. These combinations of radionuclides and chelators are preferred as they result in stable imaging agents which showed selectivity and activity in the targeted tissue (cf. examples 5 and 10).

Moreover, the RESCA chelator has the advantage that chelating at room temperature is possible (cf. example 6).

In a preferred embodiment of the first aspect the targeting moiety capable of binding GPC3 is a Fab antigen-binding fragment comprising a heavy chain antigen-binding region that comprises an H-CDR1 SEQ ID NO: 12, H-CDR2 SEQ ID NO: 13, H-CDR3 SEQ ID NO: 14 and a light chain antigen-binding region that comprises an L-CDR1 SEQ ID NO: 16, L-CDR2 SEQ ID NO: 17, L-CDR3 SEQ ID NO:18.

In another preferred embodiment of the first aspect the targeting moiety capable of binding GPC3 is a Fab antigen-binding fragment comprising a variable heavy chain domain having at least 90 %, 95 %, 98 %, 99 % or 100 % sequence identity with SEQ ID NO: 11 or a variable light chain domain having at least 90 %, 95 %, 98 %, 99 % or 100 % sequence identity with SEQ ID NO: 15.

In another preferred embodiment of the first aspect the targeting moiety capable of binding GPC3 is a Fab antigen-binding fragment comprising a variable heavy chain domain having at least 90 %, 95 %, 98 %, 99 % or 100 % sequence identity with SEQ ID NO: 11 and a variable light chain domain having at least 90 %, 95 %, 98 %, 99 % or 100 % sequence identity with SEQ ID NO: 15.

In an especially preferred embodiment of the first aspect the targeting moiety capable of binding GPC3 is a Fab antigen-binding fragment comprising the variable heavy chain domain of SEQ ID NO: 11 and the variable light chain domain of SEQ ID NO: 15.

In a further preferred embodiment of the first aspect the targeting moiety capable of binding GPC3 is a Fab antigen-binding fragment comprising a heavy chain having at least 90 %, 95 %, 98 %, 99 % or 100 % sequence identity with SEQ ID NO: 32 and a light chain having at least 90 %, 95 %, 98 %, 99 % or 100 % sequence identity with SEQ ID NO: 33.

In yet another preferred embodiment of the first aspect the targeting moiety capable of binding GPC3 is the Fab antigen-binding fragment consisting of the heavy chain of SEQ ID NO: 32 and the light of SEQ ID NO: 33.

As demonstrated in examples 5 and 10 the anti-GPC3 Fab targeting moiety reliably brought the radionuclide to the specific biological target. Moreover, examples 5 and 10 also demonstrated that the anti-GPC3 Fab fragment was the decisive component in ensuring the advantageous tumor/liver ratio for PET imaging. Hence use of the anti-GPC3 Fab fragment allowed for application and imaging of tumors on the same day as the clearance rate of the imaging agent comprising it unexpectedly fell within the time window of achieving a tumor/liver ratio of > 2 between 2-4 hours.

In a preferred embodiment of the first aspect the targeting moiety capable of binding GPC3 is a Fab antigen-binding fragment comprising a heavy chain antigen-binding region that comprises an H-CDR1 SEQ ID NO: 12, H-CDR2 SEQ ID NO: 13, H-CDR3 SEQ ID NO: 14 and a light chain antigen-binding region that comprises a L-CDR1 SEQ ID NO: 16, L-CDR2 SEQ ID NO: 17, L-CDR3 SEQ ID NO:18 the chelator is RESCA and the radionuclide is ¹⁸F or the chelator is DFO* and the radionuclide is ⁸⁹Zr.

In another preferred embodiment of the first aspect the targeting moiety capable of binding GPC3 is a Fab antigen-binding fragment comprising a variable heavy chain domain having at least 90 %, 95 %, 98 %, 99 % or 100 % sequence identity with SEQ ID NO: 11 and/or a variable light chain domain having at least 90 %, 95 %, 98 %, 99 % or 100 % sequence identity with SEQ ID NO: 15 the chelator is RESCA and the radionuclide is ¹⁸F or the chelator is DFO* and the radionuclide is ⁸⁹Zr.

In another preferred embodiment of the first aspect the targeting moiety capable of binding GPC3 is a Fab antigen-binding fragment comprising the variable heavy chain domain of SEQ ID NO: 11 and the variable light chain domain of SEQ ID NO: 15 the chelator is RESCA and the radionuclide is ¹⁸F or the chelator is DFO* and the radionuclide is ⁸⁹Zr.

Both of these imaging agents i.e. ⁸⁹Z-DFO*-Fab and ¹⁸F-RESCA-Fab showed clear tumor delineation and a tumor/liver ratio >2 within 4 hours post injection whereas other constructs did not show a tumor/liver ratio of 2 or above within this time frame (cf. examples 5 and 10, respectively).

In an especially preferred embodiment of the first aspect the targeting moiety capable of binding GPC3 is a Fab antigen-binding fragment comprising a heavy chain antigen-binding region that comprises an H-CDR1 SEQ ID NO: 12, H-CDR2 SEQ ID NO: 13, H-CDR3 SEQ ID NO: 14 and a light chain antigen-binding region that comprises an L-CDR1 SEQ ID NO: 16, L-CDR2 SEQ ID NO: 17, L-CDR3 SEQ ID NO:18, the chelator is RESCA and the radionuclide is ¹⁸F.

Surprisingly by combination of germlining and alterations of CDR sequence, it was possible to identify novel anti-GPC3 targeting moieties with promising binding properties which do not contain any lysine residue in the CDRs what makes them especially suitable as targeting moieties for radiopharmaceuticals or imaging agents which are conjugated via lysine residues. Hence, in contrast to CDR sequences of targeting moieties of the state of the art, the Fab anti-GPC3 targeting moiety described herein does not comprise any lysine residues in its CDRs. This is advantageous since conjugation of chelators, like for example RESCA, usually takes place via lysine residues of the targeting moiety e.g. a Fab and may also occur within the target binding CDR regions. Such labeling hinders the ability of the targeting moiety to bind to its target. The presence of lysine residues in the CDRs thus means a risk of increasing the non-binding fraction of an imaging agent, leading to e.g. drop in internalization rate or causing toxic side effects. At a labeling degree of approximately 10 chelators per antibody, Bell et al. (Glypican-3-Targeted Alpha Particle Therapy for Hepatocellular Carcinoma. Molecules. 2020 Dec 22;26(1):4),) observed substantial non-specific liver accumulation of their codrituzumab-based actinium conjugate [225Ac]Ac-Macropa-GC33. Most likely, this finding is due to the increased non-binding fraction of the conjugate by labeling lysines in the GC33 CDRs.

In another especially preferred embodiment of the first aspect targeting moiety capable of binding GPC3 is a Fab antigen-binding fragment comprising a variable heavy chain domain having at least 90 %, 95 %, 98 %, 99 % or 100 % sequence identity with SEQ ID NO: 11 or a variable light chain domain having at least 90 %, 95 %, 98 %, 99 % or 100 % sequence identity with SEQ ID NO: 15, the chelator is RESCA and the radionuclide is¹⁸F

In another especially preferred embodiment of the first aspect targeting moiety capable of binding GPC3 is a Fab antigen-binding fragment comprising a variable heavy chain domain having at least 90 %, 95 %, 98 %, 99 % or 100 % sequence identity with SEQ ID NO: 11 and a variable light chain domain having at least 90 %, 95 %, 98 %, 99 % or 100 % sequence identity with SEQ ID NO: 15, the chelator is RESCA and the radionuclide is¹⁸F

This is the case as the time window of the ¹⁸F -RESCA-anti-GPC3-Fab at which a tumor/liver ratio facilitating PET analysis occurred was very advantageous as it enabled same-day administration of the imaging agent and PET/CT scanning while demonstrating a low liver background and a high selectivity for GPC3 uptake which reflects target expression. Moreover, the anti-GPC3 Fab targeting moiety was shown to be robust enough for the conjugation process with RESCA.

In a further especially preferred embodiment of the first aspect the targeting moiety capable of binding GPC3 is a the Fab antigen-binding fragment comprising a heavy chain having at least 90 %, 95 %, 98 %, 99 % or 100 % sequence identity with SEQ ID NO: 32 and a light chain having at least 90 %, 95 %, 98 %, 99 % or 100 % sequence identity with SEQ ID NO: 33, the chelator is RESCA and the radionuclide is ¹⁸F.

In a highly preferred embodiment of the first aspect the targeting moiety capable of binding GPC3 is the Fab antigen-binding fragment consisting of the heavy chain of SEQ ID NO: 32 and the light chain of SEQ ID NO: 33 the chelator is RESCA and the radionuclide is ¹⁸F.

In a highly preferred embodiment of the first aspect the imaging agent is the imaging agent of formula (I) 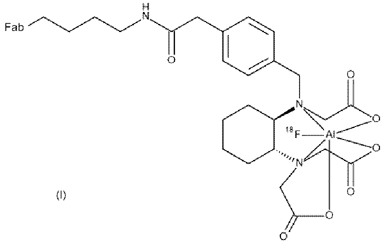 wherein Fab is the antigen-binding fragment capable of binding to GPC3 comprising a heavy chain antigen-binding region that comprises an H-CDR1 SEQ ID NO: 12, H-CDR2 SEQ ID NO: 13, H-CDR3 SEQ ID NO: 14 and a light chain antigen-binding region that comprises an L-CDR1 SEQ ID NO: 16, L-CDR2 SEQ ID NO: 17, L-CDR3 SEQ ID NO:18.

In a most highly preferred embodiment of the first aspect the imaging agent is the imaging agent of formula (I) 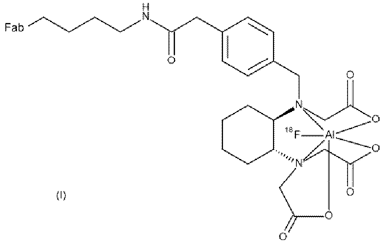 wherein Fab is the antigen-binding fragment consisting of the heavy chain of SEQ ID NO: 32 and the light chain of SEQ ID NO: 33.

### Method of producing the imaging agent (Second Aspect)

In a second aspect what is decribed herein relates to a method for producing an imaging agent of the first aspect.

### Kit comprising the imaging agent (Third Aspect)

In a third aspect what is decribed herein relates to a kit comprising the imiagin agent of the first aspect and instructions for use.

### Uses (Fourth Aspect)

In a fourth aspect the antibodies fragments and imaging agents described herein may be used for determining if GPC3 expressing cells such as differentiating hepatocellular carcinoma cells exist e.g. also in diagnostic PET imaging.

### EXPERIMENATL SECTION

### Abbreviations

The following table lists the abbreviations used herein. Other abbreviations have their meanings customary *per se* to the skilled person.
- ²²⁵Ac: actinium-225
- Ac-225: actinium-225
- DMA: N,N-dimethylacetamide
- DMSO: dimethyl sulfoxide
- EtOH: ethanol
- FPLC: fast protein liquid chromatography
- GPC3: glypican-3
- HPLC: high performance liquid chromatography
- iTLC: instant thin layer chromatography
- IRF: immunoreactive fraction
- mAb: monoclonal antibody
- min: minutes
- MS: mass spectrometry
- NaCl: sodium chloride
- nm: nanometer
- nmol: nanomole
- PBS: phosphate buffered saline
- PET: positron emission tomography
- RAC: radioactive concentration
- RCP: radiochemical purity
- SEC: size exclusion chromatography
- TAC: targeted actinium conjugate (Ac-225 labelled ACC)
- TFA: trifluoroacetic acid
- TOF: time of flight
- UV: ultraviolet

### Example 1 Generation of anti-GPC3 targeting moieties and reference compounds

All antibodies were expressed in HEK293 cells using standard transient transfection procedures and purified from the cell culture supernatant via Protein-A and size exclusion chromatography. In some examples, prior art antibody sequences such as those of TPP-14890 (Codrituzumab) were randomly altered and/or subjected to sequence germlining and further alterations were introduced. A few of the resulting antibodies showed improved or mostly unchanged behavior. This was unexpected as Nakano et al. (Nakano et al., Anti-glypican 3 antibodies cause ADCC against human hepatocellular carcinoma cells. Biochem Biophys Res Commun. 2009 Jan 9;378(2):279-84) who have described the humanization and optimization of GC33 giving rise to Codrituzumab showed that even substitution of single amino acids led to complete loss of binding (e.g. N33D in CDR-L1). Thus, the expectation was that little that more of alteration could be performed without complete loss of beneficial properties. One example with improved or mostly unchanged characteristic was surprisingly TPP-29537 despite a very high number of mutations in both VH and VL as compared to Codrituzumab. TPP-29537 was characterized with regard to monovalent binding affinity (KD), surface plasmon resonance (SPR) on human and cynomolgus GPC3, cellular binding (EC50) to CHO cells transfected with either human or cynomolgus GPC3 by flow cytometry, as well as melting temperature of the Fab (data not shown) and chosen for the development of an imaging agent.

The VHH anti-GPC3 targeting moiety (SEQ ID NO: 31) was produced by expression in HEK293 cells using standard transient transfection procedures and purified from the cell culture supernatant via Ni-NTA affinity chromatography followed by size exclusion chromatography. The anti-GPC3 targeting moiety formats scFv₂, Fab, Fab-isotype, Fab₂, and Fab₂-isotype were produced by enzymatic digestion of whole IgG (TPP-29537 SEQ ID NO. 37 and SEQ ID NO. 38) or scFv-Fc (TPP-30783 SEQ ID NO: 41) using Papain or IdeS proteases followed either by Ni-NTA affinity chromatography to remove His-tagged protease and/or size exclusion chromatography and/or ProteinA affinity chromatography. Anti-GPC3 IgG and scFv-Fc used as substrates for the enzymatic digests were produced by expression in HEK293 cells using standard transient transfection procedures and purified from the cell culture supernatant via Protein-A affinity chromatography followed by size exclusion chromatography.

**Table 1**

| **Targeting moiety** | **SEQ ID NO** | **Size** (kDa) |
|---|---|---|
| VHH | 31 | 15 |
| scFv₂ | 40 | 56 |
| Fab (APP-10500) | 32 and 33 | 48 |
| Fab isotype from TPP-754 | 34 and 35 | 47 |
| Fab₂ | 36 and 37 | 98 |
| Fab₂ isotype from TPP-754 | 38 and 39 | 96 |

### Example 2 Conjugation of anti-GPC3 targeting moieties to DFO*

The various anti-GPC3 targeting moieties were conjugated to 5,11,16,22-Tetraazahexacosanediamide,N1-[5-(acetylhydroxyamino)pentyl]-N26,5,16-trihydroxy-N26-[5-[[[(4-isothiocyanatophenyl)amino] thioxomethyl]amino]pentyl]-4,12,15,23-tetraoxo-(p-Phe-NCS-DFO* (DFO*), ABX advanced chemical compounds, 7272) dissolved in DMSO to 10 mg/ml. The pH of the anti-GPC3 targeting moieties was adjusted to 9 with 1M carbonate buffer. p-Phe-NCS-DFO* was added to the anti-GPC3 targeting moieties and the mixture was incubated on a thermomixer shaking at 1 hour at 37°C. The conjugate was purified using FPLC (column: Superdex^{®} 200 Increase 10/300 GL column, code: 28990944, running buffer: 0.5 M HEPES). The monomeric purity of the DFO*- anti-GPC3 targeting moieties conjugates was measured by SEC-UV. CAR was determined by SEC-MS using positive electrospray ionization and the following equation: CAR = Sum(n*An)/Sum An, where n equals the number of chelators and An equals the intensity of the conjugate with n chelators. Results are given in Table 2.

### Example 3 Chelating of DFO*-anti-GPC3 targeting moieties with Zirconium-89

Zirconium-89 in 1M oxalic acid was added to DFO*- anti-GPC3 targeting moieties in 1 M HEPES, pH 7.3, and labeled with a specific activity of 34 MBq/mg and a RAC of 103 MBq/mL. After incubation for 30 minutes at 37°C, the sample was purified by PD-10 column according to manufacturer's instructions (Cytiva, 17085101) and buffer exchanged to formulation buffer (10 mM L-Histidine, 130 mM Glycine, 5% (m/v) Sucrose in water for injection) and diluted to RAC of 24 MBq/mL. The results are shown in Table 2.

**Table 2**

| **Compound** | **SEQ ID NO** | **CAR** | **Conjugation** (DFO*) | **Chelation** (HPLC, iTLC > 95%) | **Stability 24h serum** (HPLC, iTLC > 95%) |
|---|---|---|---|---|---|
| VHH (nanobody) | 31 | 1.1 | Yes | Yes | Yes |
| scFv₂ | 40 | 0.6 | Yes | Yes | Yes |
| Fab | 32,33 | 1.1 | Yes | Yes | Yes |
| Fab isotype | 34,35 | 1.3 | Yes | Yes | Yes |
| Fab₂ | 36,37 | 1.0 | Yes | Yes | Yes |
| Fab₂ isotype | 38,39 | 1.0 | Yes | Yes | Yes |

### Example 4 Immunoreactive fraction assay of ⁸⁹Zr- DFO*-anti-GPC3 targeting moieties

Immunoreactive fraction (IRF) determination was used to evaluate the binding properties of the radiolabeled constructs of Example 3. Shortly after completion of the radiolabeling, the binding of the radiolabeled constructs of Example 3 to GPC3 peptide-coated M-270-carboxy Dynabeads was determined. In short, 0.36-800 µg beads were placed in 2 mL Eppendorf tubes in 90 µL buffer (PBS/3% BSA). The radiolabeled constructs of Example 3 were diluted to 5 Bq/µL in buffer and 10 µL were added to each bead-containing tube. The samples were run in triplicate. The binding lasted for 60 min while shaking the tubes on a Thermomixer (Eppendorf) at 750 rpm at room temperature. After completion of the binding step, a magnet (DynaMag-2) was used to separate the beads from half of the supernatant. The radio activities in the supernatant and beads of each sample were determined by using a high purity Germanium detector (Ortec). Total binding was defined as [(activity in beads - activity in supernatant)/ (total activity)] x 100. It was demonstrated that active and intact ⁸⁹Z- DFO*-anti-GPC3 construct could be provided (data not shown). In addition, binding of the radiolabeled ⁸⁹Zr-DFO*-anti-GPC3-Fab to the GPC3 expressing cell line (HepG2) was determined. In short, 25 000- 25 000 000 cells were placed in 2 mL Eppendorf tubes in 200 µL buffer (PBS/3% BSA). The ⁸⁹Zr- DFO*-anti-GPC3-Fabs were diluted to 5 Bq/µL in buffer and 10 µL were added to each cell - containing tube. The binding lasted for 60 min while shaking the tubes on a Thermomixer (Eppendorf) at 750 rpm at room temperature. After completion of the binding step, the cells were centrifuged to separate cells from half of the supernatant. The radio activities in the supernatant and cell pellet of each sample were determined by using a high purity Germanium detector (Ortec). Total binding was defined as [(activity cell pellet - activity in supernatant)/ (total activity)] x 100. The result is shown in table 3 below.

**Table 3**

| | **MAX % IRF** |
|---|---|
| ⁸⁹Zr-DFO*-anti-GPC3-Fab | **67.4 %** |

Thus, it was demonstrated that active and intact ⁸⁹Z- DFO*-anti-GPC3-targeting moieties could be provided.

### Example 5 Animal experiments using ⁸⁹Zr- DFO*-anti-GPC3 targeting moieties

Eight to 10 weeks old female athymic Rj:NMRI-*Foxn1*^{*nu*/*nu*} mice (Janvier, France) were allowed to acclimatize for 1 week prior to experimentation and were housed in groups of 6-8 mice. Xenograft models of hepatocellular carcinoma were established by subcutaneous inoculation of a 100 µL suspension containing 1 x 10⁶ HEP-3B cells in a 1:1 PBS/Matrigel mixture. Mice were used for experimentation 2 to 3 weeks after inoculation, when tumors had grown to >50 mm³. The following constructs were used: ⁸⁹Zr- DFO*-VHH (nanobody), ⁸⁹Zr- DFO*-scFv₂ , ⁸⁹Zr- DFO*-Fab (APP-10500), ⁸⁹Zr-DFO*-Fab₂ and a ⁸⁹Zr- DFO*-non-binding Fab (APP-277). The constructs were injected as a slow bolus into the tail vein of 2-5 animals per construct at 200 MBq/mg. At the relevant time points (cf. Table 4) organs of interest and tumor were collected, weighed, and measured in a calibrated Hidex automated gamma counter standard (Hidex). Counts of known standards were used to convert counts into injected dose. Tissue radioactivity was expressed as percentage injected dose per gram (%ID/g) and converted into tumor / liver ratio where applicable. Results are shown in Table 4.

**Table 4**

| **⁸⁹Zr- DFO*- anti-GPC3 targeting moieties** | **SEQ ID NO** | **Tumor to liver ratio** | |
|---|---|---|---|
| | | 1h p.i.. | 4h p.i.. |
| VHH (nanobody) | 31 | ~2 | >2 |
| scFv₂ | 40 | < 1 | ~ 1 |
| Fab (APP-10500) | 32,33 | < 2 | >2 |
| Fab₂ | 36,37 | < 1 | < 1 |
| Non binding Fab | 34,35 | ~ 0 | < 1 |

Both ⁸⁹Zr- DFO*-VHH and ⁸⁹Zr- DFO*-Fab showed tumor/liver ratio >2 within 4 hours p.i., whereas the other constructs did not show a tumor/liver ratio of 2 or above within this time frame. Hence of the possible compounds for PET imaging during a favorable time span the ⁸⁹Z- DFO*-VHH and ⁸⁹Z- DFO*-Fab were demonstrated to be the most suitable compounds. For the Fab this was surprising as the Fab of CN117247454 did not reach a tumor/liver ratio of above 2 (cf. Fig. 8 of CN117247454).

### Example 6 Conjugation of anti-GPC3-Fab to RESCA chelator and analysis

RESCA-tetrafluorophenyl ester ((±)-H₃RESCA-TFP (Chematech) was dissolved in DMSO to 10 mg/ml. RESCA (RESCA-TFP) was added to the anti-GPC3-Fab (APP-10500) generated as described above in Example 1 and the mixture was incubated on a thermomixer shaking for 2.5 hours at 37°C. The conjugate was purified using FPLC (column: Superdex^{®} 200 Increase 10/300 GL column, code: 28990944, running buffer: 0.1 M sodium acetate, 0.1 M NaCl). The monomeric purity of the RESCA-anti-GPC3-Fab conjugate was measured by SEC-UV.

### 6.1 CAR ratio

The Chelator-to-antigen-targeting-moiety ratio here Chelator-to-Fab ratio was determined by SEC-MS (Water Acquity HPLC connected to Waters XEVO TOF; running buffer: 50/50/0.1 (v/v/v) ACN/Water/TFA; flow rate: 0.06 mL/min, column for on-line desalting: Waters Acquity BEH SEC, 1.7 µm, 2.1 x 150 mm; ESI+ mode) by using 15 MS peak heights in percentage of major peak height for the components Fab, Fab + 1 chelator, Fab + 2 chelators, Fab + 3 chelators etc. and using the formula CAR = Sum(n*An)/Sum An, where n equals the number of chelators and An equals the intensity of the Fab conjugate with n chelators. Purity and concentration of the RESCA-anti-GPC3-Fab was determined by SEC-UV (Agilent 1260 Infinity HPLC system, running buffer: 10% isopropanol/2 mM EDTA/150 mM NaCl in PBS; flow rate: 0.3 mL/min, column: Waters Acquity BEH SEC, 1.7 µm - 200Å, 4.6 x 300 mm, detection: UV at 280 nm). The method separates higher aggregates, dimers, fragments and free chelator from the Fab conjugate monomer. Results are shown in Table 5.

**Table 5**

| Compound | CAR | Monomeric purity | Concentration (mg/ml) |
|---|---|---|---|
| RESCA-anti-GPC3-Fab (APP-10500) | 1.1 | 99.9 % | 1.40 |
| RESCA-anti-GPC3-Fab (APP-10500) | 1.0 | 99.9 % | 4.83 |
| Isotype-RESCA (APP-277) | 0.84 | 99.9 % | 2.86 |

The results showed that the RESCA-anti-GPC3-Fab conjugate was monomeric and was binding. This was not predictable as binding of RESCA in the setting was via the lysine(s) of the anti-GPC3-Fab and could have led to multimers.

### 6.2 ELISA

An ELISA was carried out as follows: GPC3 antigen was immobilized on ELISA plate by absorption. The plate was washed (PBS with 0.1% Tween 20) and incubated with blocking buffer (PBS + 3% BSA) for 2 h ± 1 h to reduce unspecific binding. Fab and RESCA-anti-GPC3-Fab conjugate was diluted in assay buffer (PBS with 0.05% Tween 20 + 1.5% BSA). The blocking buffer was discarded, and Fab, Fab conjugate and controls were applied in triplicates to the plate according to a plate set-up defined in each protocol. After incubation for 1 h ± 30 min at RT, the plate was washed. A horse radish peroxidase (HRP)-conjugated secondary antibody binding specifically to the Fab (anti-human Kappa -HRP) was added to the plate. After incubation for 1 hour ± 30 min at RT, the plate was washed. A chromogenic substrate (2,2'-azino-bis (3-ethylbenzothiazoline-6-sulphonic acid) (ABTS)), for HRP leading to the formation of a green color was added to the plate and used for detection. The absorbance of the colored reaction product was measured with an absorbance plate reader, e.g. EnVision 2103 Multilabel Reader from Perkin Elmer, or iMark Microplate Absorbance Reader from Bio-Rad. Absorbance against the log of the RESCA-GPC3-Fab concentration was plotted on the GraphPad Prism software. A dose stimulation fit model with "log (agonist) vs response variable slope (four/three parameters)" was used to calculate the concentration necessary to reach 50% of the maximum Absorbance (EC50). Results are shown in the Table 6.

**Table 6**

| **Compound** | **EC50** |
|---|---|
| Fab-RESCA CAR 1.0 | 3.336 |
| Fab non-conjugated | 2.956 |

The ELISA results showed that conjugation with Resca had a negligible effect on the affinity of the anti-GPC3-Fab targeting moiety.

### 6.3 SPR

Surface Plasmon Resonance (SPR) measurements were performed to assess the influence of RESCA-conjugation on target binding affinity. Non-conjugated anti-GPC3-Fab (APP-10500) was compared to GPC3-Fab-RESCA-conjugates (APP-10500) with DARs of ~1 , ~2 and ~4, respectively. Anti-human Fab capture antibody (Human Fab Capture Kit; #28958325; Cytiva) was covalently coupled to a CM5 chip using the amine coupling kit (#BR100050; Cytiva) following the manufacturer's instructions to a density of ~ 9.000 RU. Naked Fab and its respective conjugates were capture to a ligand level between 50 and 90 RU. Analyte (human GPC3; R&D Systems, #2119-GP) was injected in multicycle kinetics mode in a titration series from 0.039 nM 100 nM (2-fold dilution). The experiments were run in HBS-EP+ (Cytiva). Association time was set to 180 s, the dissociation time to 900 s and the flow rate was set to 30 µl/min. Obtained data were double referenced (subtraction of the reference cell and the 0 nM concentration signal) and fitted to a 1:1 Langmuir binding isotherm (RI set to constant) using the Biacore T200 evaluation software. Results are shown in Table 7

**Table 7**

| **Compound** | **KD (M)** |
|---|---|
| Fab-RESCA CAR 1.1 | 3,3E-09 |
| Fab-RESCA CAR 2.2 | 3,2E-09 |
| Fab-RESCA CAR 4.3 | 3,4E-09 |
| Fab non-conjugated (APP-10500) | 3,3E-09 |

As can be seen from the kinetic data, conjugation with RESCA-chelator did not change the binding affinity (KD) of the anti-GPC3-Fab targeting moiety to the target GPC3 compared to the anti-GPC3-Fab targeting moiety without RESCA i.e. the naked Fab APP-10500. There was no change in binding affinity with increasing CAR of the Fab.

### Example 7 Conjugation and chelating of anti-GPC3-Fab to NOTA chelator

For conjugating the anti-GPC3-Fab to 1,4,7-Triazacyclononane-1,4,7-triacetic acid (NOTA, cat# B605, Macrocyclics), CAS 1206475-68-4 the pH of the anti-GPC3-Fab was adjusted to 9 with 1M carbonate buffer. p-SCN-Bn-NOTA was dissolved in DMA to 10 mg/ml and added to the pH adjusted anti-GPC3-Fab. The mixture was incubated on a thermomixer shaking at 1 hour at 37°C. The conjugate was purified using FPLC (column: Superdex^{®} 200 Increase 10/300 GL column, code: 28990944, running buffer: 0.1 M sodium acetate, 0.1 M NaCl). The monomeric purity of the conjugate was measured by SEC-UV. CAR was determined by SEC-MS using positive electrospray ionization and the following equation: CAR = Sum(n*An)/Sum An, where n equals the number of chelators and An equals the intensity of the Fab conjugate with n chelators. The monomeric purity of the NOTA-anti-GPC3-Fab conjugate was measured by SEC-UV. In the next step the NOTA-anti-GPC3-Fab conjugate was to be chelated with ¹⁸F adapted from Li et al.2020. However, all intact NOTA-anti-GPC3-Fab conjugate was completely lost during the chelating step. This was unexpected as the smaller peptides used by Li et al. 2020 could be radiolabeled with Nota using the Al[18F]F chelation approach.

### Example 8 Chelation of RESCA-anti-GPC3-Fab with ¹⁸F

Cyclotron-produced [¹⁸F]fluoride in water was purified through a carbonate QMA cartridge preconditioned with 0.4 M sodium bicarbonate. The eluted [¹⁸F]fluoride in sodium bicarbonate was neutralized with glacial acetic acid. The desired amount of radioactivity was added to 0.1 M sodium acetate buffer (pH 4) containing aluminum chloride (AlCl₂) at a molar ratio of 50 nmol/MBq. This reaction was incubated for 5 minutes at room temperature. Next, the resulting [¹⁸F]AlF₂ solution was transferred to a new reaction vial containing the RESCA-anti-GPC3-Fab(APP-10500) conjugate of Example 6 in a 0.1 M sodium acetate buffer (pH 4), and the reaction mixture was incubated for 15 minutes at 37°C. ¹⁸F -RESCA-anti-GPC3-Fab was purified using either a 30 kDa microcon centrifuge filter (Merck) or a PD-10 desalting column, and reconstituted in the formulation buffer consisting of 50 mM L-methionine in Saline. Radiochemical purity was determined by using iTLC-silica gel strips analyzed by miniGITA Star scanner (Raytest) with 0.9% saline as mobile phase. Additional analysis, including compound integrity, was determined by SEC-HPLC (Agilent 1260 Infinity HPLC system), using a Waters Acquity BEH SEC colum ( 1.7 µm - 200Å, 4.6 x 300 mm) and a radiodetector. Mobile phase consisted of 10% isopropanol /150 mM NaCl in PBS at a flow rate of 0.3 mL/min.

### Example 9 Immunoreactive fraction (IRF) of ¹⁸F -RESCA-anti-GPC3-Fab

Shortly after completion of the radiolabeling of Example 8, the binding was assessed by measuring the fraction of ¹⁸F-RESCA-anti-GPC3-Fab(APP-10500) bound to GPC3 antigen coated magnetic beads (Thermo Fisher Scientific). In short, beads were placed in 2 mL Eppendorf tubes in 90 µL buffer (PBS/0.1% Tween 20/1% BSA). The ¹⁸F-RESCA-anti-GPC3-Fab were diluted to 5 Bq/µL in buffer and 50 Bq were added to each bead-containing tube. The binding lasted for 60 min while shaking at 750 rpm at room temperature. After completion of the binding step, magnet (DynaMag-2) was used to separate the beads from half of the supernatant. The radio activities in the supernatant and in the beads of each sample were measured using a high purity Germanium detector (Ortec). Total binding was defined as [(activity in beads - activity in supernatant)/ (total activity)] x 100.

The results shown in Table 8 demonstrated that the ¹⁸F-RESCA-anti-GPC3-Fab was intact and capable of binding.

**Table 8**

| | **MAX % IRF** |
|---|---|
| ¹⁸F- RESCA-anti-GPC3-Fab (APP-10500) | ~80% |
| ¹⁸F-RESCA- Isotype | ~ 1 % |

### Example 10 Animal experiments using ¹⁸F-RESCA-anti-GPC3-Fab

Eight to 10 weeks old female athymic Rj:NMRI-*Foxn1*^{*nu*/*nu*} mice (Janvier, France) were allowed to acclimatize for 1 week prior to experimentation and were housed in groups of 6-8 mice. Xenograft models of hepatocellular carcinoma were established by subcutaneous inoculation of a 100 µL suspension containing 1 x 10⁶ HEP-3B cells in a 1:1 PBS/Matrigel mixture. Mice were used for experimentation 2 to 3 weeks after inoculation, when tumors had grown to >50 mm³. ¹⁸F-RESCA-anti-GPC3-Fab(APP-10500) or a non-binding control were injected as a slow bolus into the tail vein of 3 animals per construct at 200 MBq/mg. PET/CT imaging was performed 2 h (n=1), 4 h (n=3), 24 h (n=3), 72 h (n=3) and 168 h (n=2) post injection. The imaging acquisitions were performed by MILabs Vector6 PET/SPECT/CT scanner using HE-UHR-RM collimator, 4-mouse bed, total acquisition time of 60 minutes, spiral acquisition mode and fixed field of view, determined during the first acquisition. The PET imaging of ¹⁸F-RESCA-anti-GPC3-Fab showed clear tumor delineation and a tumor/liver ratio of >2 after 3 hours. This finding was confirmed using the cut and count protocol described above in example 5 leading to the data shown below in Table 9. In addition, it was found that the concentration in the liver was comparable to that in blood and intestine and considerable higher than in muscle.

**Table 9**

| | **Tumor to liver ratio 3h p.i. (cut & count)** |
|---|---|
| ¹⁸F-RESCA-anti-GPC3-Fab (0.3 nmol/3 nmol) | >2 |
| ¹⁸F-RESCA-anti-GPC3-Fab (3 nmol) | ~ 1 |

Thus, the time window of the ¹⁸F-RESCA-anti-GPC3-Fab at which a tumor/liver ratio facilitating PET analysis occurred was very advantageous as it enabled same-day administration of the imaging agent and PET/CT scanning while demonstrating a low liver background and a high selectivity for GPC3.

Moreover, ex-vivo, two doses ¹⁸F-RESCA-anti-GPC3-Fab (0.3 and 3 nmol / mouse) showed similar tumor uptake, while the non-binding (NB) Fab (¹⁸F-RESCA-nb-anti-GPC3-Fab) had reduced tumor uptake indicating that the tumors were not saturated by the higher dose also used in example 10.

### Example 11 Experiments on the effect of lysine residues in CDRs using TPP-29537

The following section describes experiments disclosed in unpublished PCT/EP2024/078685 showing that TPP-29537 which has the same CDRs as the Fab targeting moiety comprising an H-CDR1 SEQ ID NO: 12, H-CDR2 SEQ ID NO: 13, H-CDR3 SEQ ID NO: 14 and a light chain antigen-binding region that comprises an L-CDR1 SEQ ID NO: 16, L-CDR2 SEQ ID NO: 17, L-CDR3 SEQ ID NO:18 has beneficial properties compared to prior art antibody TPP-14890 which has lysine in its CDRs.

### 11.1 Internalization

Internalization is a process in which a targeting moiety such as an antibody, after binding to its specific tumor target, is taken up into the tumor cell. Internalization is not a prerequisite for the effect of TACs or an imaging agent, since the radioactive radiation can kill the target cell after binding of the TAC to the receptor even without internalization. However, internalization can also be beneficial here as it may contribute to improved tumor retention.

*Internalization of directly CypHer5E labelled anti-GPC3 antibodies.*

In a first step, the antibodies TPP-29537 and TPP-14890 were directly labelled at a concentration of 1 mg/ml with the pH sensitive dye CypHer5E-NHS-Ester (Cytiva, PA 15401), which was coupled to lysine residues of the antibodies according to the instruction of the manufacturer.

After labelling the dye to protein ratio was estimated to be 21.5 for TPP-29537 and 16.4 for TPP-14890.

HepG2 cells (20,000 cells/well) were plated onto Greiner µCLEAR 96-well microtiter plates (Greiner, #655090) in 100 µl EMEM medium (ATCC, 30-2003) containing 10% FCS. After an overnight incubation at 37°C and 5% CO₂, 10 µg Hoechst 33342 (Invitrogen, #H3570) staining solution in 10 µl medium was added to each well and incubated for 10 min in the dark. Next, the medium was removed and replaced by 90 µl fresh medium as well as the respective antibody of interest (10 µL per well, final concentration of 2 µg/mL). Cells were then incubated at 37°C and 5 % CO₂ in the incubator. At 0.15-, 1-, 3-, 5-, and 24-hours pictures were taken by the high content analysis system ImageXpress (Molecular Devices). Images were analyzed using the software MetaXpress (Molecular Devices) applying the Transfluor module (settings "Vesicles 5_10_500" and "Nuclear stain 6_32_100"). The vesicle area per cell was calculated for the individual antibodies and values obtained at 5 hours were > 70% vesicle area per cell for TPP-29537 and < 20% vesicle area per cell for TPP-14890.

*Internalization of Fabfluor labelled antibodies.*

HepG2 cells (20,000 cells/well) were plated onto Corning BioCoat µClear 96-well MTP (356640) in 100 µl EMEM medium (ATCC, 30-2003) containing 10% FCS and incubated over night at 37°C and 5% CO₂. The next day, 5 µl of a 10 µg/ml solution of Incucyte^{®} human Fabfluor-pH antibody labeling reagent (Satorius, #4722), a dye labeled Fab fragment binding to Fc containing proteins, was mixed with 5 µl of 20 µg/ml solutions of individual test antibodies for 20 min at room temperature. Then, 90 µl EMEM medium was added and the mixture incubated for 5 additional minutes on a shaker. In addition, 10 µg Hoechst 33342 (Invitrogen, #H3570) staining solution in 10 µl medium was added to each well of the plate containing the cells and incubated for 10 min in the dark. Then, the medium from the tissue culture plate was removed and replaced by 90 µl of the antibody - Fabfluor mixtures. Cells were then incubated at 37°C and 5 % CO₂ in the incubator. At 0.15-, 1-, 3-, 5, and 24-hours pictures were taken by the high content analysis system ImageXpress (Molecular Devices). Images were analyzed using the software MetaXpress (Molecular Devices) applying the Transfluor module (settings "Vesicles 5_10_500" and "Nuclear stain 6_32_100"). The vesicle area per cell was calculated for the individual antibodies and values obtained at 5 hours were within the same range for both antibodies (data not shown).

It became obvious that TPP-14890 showed lower internalization compared to TPP-29537 using direct labeling of the antibodies with CypHer5E via covalent labeling of lysine residues. In contrast using Fabfluor non-covalent labeling, both anti-GPC3 antibodies show comparable internalization rates. These differences in the internalization rates can be explained by the fact that direct labeling using lysine coupling chemistry also leads to labeling of one or more of overall four lysine residues within the CDRs of TPP-14890 which results in an affected binding and therefore in a lowered internalization rate. As antibody TPP-29537 and Fab APP-10500 contain no lysine residue within the CDRs binding and internalization rates are not affected. This example showed that TPP-29537 and hence Fab APP-10500 are more suitable for applications based on lysine chemistry conjugation, e.g., use as part of an imaging agent as described herein than prior art antibodies comprising lysine residues within their CDRs, as e.g., antibody TPP-14890.

### 11.2 Cell surface binding to different cell lines

Cell surface binding of TPP-14890, TPP-29537 and isotype control was investigated by flow cytometry using different cultured cells. The cell lines SU-DHL-1, HFF-1, HepG2, Hep3B2, PLC/PRF/5 were cultured in RPMI + 10% FCS, DMEM + 15% FCS, RPMI + 10 % FCS, MEM + 10% FCS and MEM + 10% FCS respectively. Primary human cardiac fibroblasts (HCF) and primary human pulmonary fibroblasts (HPF) were purchased from Promocell and cultured according to the manufacturer's instructions. For surface staining cells were detached using non enzymatic dissociation solution (Gibco #13151-014). Cells were washed twice in ice cold FACS buffer (PBS -/- with 3% FCS from Sigma #F2442). All subsequent steps were conducted on ice to prevent antibody internalization. 200,000 cells in 100µl were transferred per well to a V-shaped 96 well plate. Cells were stained with Alexa Fluor 488 labeled TPP-14890, Alexa Fluor 488 labeled anti-GPC3 antibody TPP-29537 or FITC labeled isotype control antibody. Antibodies were directly labeled using A10235, Alexa Fluor 488 from Invitrogen which couples to primary amino groups present on lysine residues. Degree of labeling was determined following the manufacturer's instructions. TPP-14890 had a degree of labeling (DOL) of 6.07; TPP-29537 DOL=7.22 and a control of DOL=3.4. All antibodies were diluted in FACS buffer to reach a final concentration of 10 µg/mL. Staining was done for 30 min at 4°C shaking on a Titramax100 in the dark. Cells were washed with PBS-/- and centrifuged at 800g for 2 min at 4°C. Subsequently the supernatant was carefully removed. Cells were resuspended in FACS buffer containing 1:1000 Sytot Blue (Invitrogen S34857). Cells were measured on a BD Biosciences FACSCanto^{™} II Flow Cytometry System and analyzed using the FlowJo_v10.6.1 software (BD Biosciences). Living, single cells were gated using forward scatter (FSC), side scatter (SCC) and Pacific Blue gating. Along with the cells MESF beads (Quantum 488C, Lot.: 14506) were measured. The measured fluorescent signals were converted into an ABC-value (Antibody Binding Capacity) with help of the MESF beads following the manufacturer's instructions. After calculation of ABC-values the isotype control ABC values were subtracted from the values of the target antibodies.

**Table 10: ABC values for various cell lines determined using TPP-14890 or TPP-29537**

| | **Sample Name** | **ABC values** |
|---|---|---|
| | | Target - Isotype ctrl |
| **TPP-14890** | HFF-1 | 0,0 |
| | SU-DHL-10 | 0,0 |
| | PLC/PRF/5 | 0,0 |
| | HepG2 | 41734,3 |
| | Hep3B2 | 9750,7 |
| | HCF | 0,0 |
| | HPF | 0,0 |
| **TPP-29537** | HFF-1 | 0,0 |
| | SU-DHL-10 | 0,0 |
| | PLC/PRF/5 | 14495,1 |
| | HepG2 | 857064,2 |
| | Hep3B2 | 192253,2 |
| | HCF | 0,0 |
| | HPF | 0,0 |

It becomes obvious that in case of HepG2 and Hep3B2 the Alexa Fluor 488 labeled TPP-29537 detects around 20-fold higher ABC value as compared to the Alexa Fluor 488 labeled TPP-14890. For PLC/PRF/5 the Alexa Fluo r488 labeled TPP-29537 detects an ABC value of 14495,1 whereas the Alexa Fluor 488 labeled TPP-14890 is not able to detect any GPC3 on this cell line having lower amounts of GPC3 on its surface. For the cell lines with ABC = 0, the calculated ABC value for the GPC3 antibody was lower than for the isotype control antibody in that respective cell line. An ABC value of 0 indicates no or very low GPC3 protein expression. These differences in ABC values can be explained by the fact that direct labeling with Alexa Fluor 488 using lysine coupling chemistry also leads to labeling of one or more of overall four lysine residues within the CDRs of the prior art antibody TPP-14890 which results in an affected binding to GPC3. As anti-GPC3 antibody TPP-29537 and hence Fab APP-10500 contain no lysine residue within the CDRs binding and resulting ABC values are not affected.

### 11.3 Comparison of GPC3-TACs in immunoreactive fraction assay (IRF)

Binding of Ac-225-Macropa-TPP-29537 and Ac-225-Macropa-TPP-14890 to GPC3-coated M-270-carboxy Dynabeads (Thermo Fisher Scientific) was determined by immunoreactive fraction (IRF) assay. IRF is assessed by measuring the fraction of radiolabeled antibodies bound to magnetic antigen-coated beads, compared to the unbound fraction still in the supernatant. Binding was assessed directly after radiolabeling.

In brief, a decreasing amount of magnetic beads from 400 to 0.1 µg was placed in 2 mL Eppendorf tubes and resuspended in 50 µL buffer (PBS/ 3% BSA). The radiolabeled compounds were diluted to 5 Bq/µL in buffer and added to each bead-containing tube (50Bq total). Incubation lasted for 60-90 min while shaking the tubes on a Thermomixer (Eppendorf) at 750 rpm at room temperature. After completion of the binding step, 40 µL of buffer was added to each tube and a magnet (DynaMag-2) was used to separate the beads from the supernatant. The radioactivity in the supernatant and on the beads of each sample was determined using a high purity Germanium detector (Ortec) after at least 6 hours, to allow daughter nuclides to be in secular equilibrium. Total binding was defined as [(activity in beads - activity in supernant)/(total activity)]x100. The unspecific binding was obtained from the same calculation from blocked samples, and specific binding was defined as [total binding - unspecific binding]. Results are shown in Table 11.

**Table 11: CAR, EC50 (µg beads) and MAX % IRF of Ac-225-Macropa-TPP-29537 and Ac-225-Macropa-TPP-14890.**

| | ***CAR*** | ***EC50*** [µg beads] | **MAX % IRF** |
|---|---|---|---|
| Ac-225-Macropa-TPP-29537 | 0.5 | 17.26 | 95 |
| | 11 | 15.02 | 94 |
| Ac-225-Macropa-TPP-14890 | 0.7 | 18.97 | 96 |
| | 10 | 35.12 | 95 |

For Ac-225-Macropa-TPP-29537 which has the same CDRs as Fab APP-10500 comparable EC50 and max % IRF were determined for both low and high CAR conjugates, whereas for the high CAR Ac-225-Macropa-TPP-14890 conjugate comprising the prior art anti-GPC3 antibody TPP-14890 there was a clear decrease in EC50 value compared to the low CAR version, indicating an impaired binding potency to human GPC3 peptide.

### 11.4 In vitro cytotoxicity

In *vitro* cytotoxicity of Ac-225-Macropa-TPP-14890 (TPP-14890-TAC) CAR 0.7, Ac-225-macropa-TPP-14890 CAR 10, Ac-225-Macropa-TPP-29537 (TPP-29537-TAC) CAR 0.5 and Ac-225-Macropa-TPP-29537 CAR 11 was determined in Human Hepatocellular Carcinoma (HCC) cell lines expressing different levels of GPC3. HepG2 and Hep3B2.1-7 cells were seeded at an appropriate cell density in the cell media MEM + 10% FCS. HepG2 cells have higher GPC3 expression on the cell surface than Hep3B2.1-7 cells. One day after seeding the compounds at the specific activity of 2 MBq/mg and radioactive concentration of 5 kBq/ml were titrated in parallel with a radiolabeled isotype control. Cells were incubated for 6 days and subsequentially cell viability was determined using Hoechst and PI staining and analyzed on Operetta CLS High Content Analysis System (Perkin Elmer). Resulting IC₅₀ values are shown in Table 12.

Table **13** 113 shows the Isotype/Targeting ratio of the different TACs (a higher Isotype/Targeting ratio indicates higher specific cytotoxicity).

**Table 12: IC₅₀ values of TPP-14890-TAC and TPP-29537-TAC with low and high CARs**

| **Cell line** | **IC₅₀ values (kBq/mL)** | | | | |
|---|---|---|---|---|---|
| | **TPP-14890-TAC CAR 0.7** | **TPP-29537-TAC CAR 0.5** | **TPP-14890-TAC CAR 10** | **TPP-29537-TAC CAR 11** | **Isotype CAR 0.7** |
| **HepG2** | 0.0065 | 0.0041 | 0.0158 | 0.0068 | 1.003 |
| **Hep3B2.1-7** | 0.0178 | 0.0110 | 0.0673 | 0.0157 | 0.2998 |

**Table 13 1: IC₅₀ Ratio (Isotype/Targeting) of TPP-14890-TAC and TPP-29537-TAC with low and high CARs**

| **Cell line** | **IC₅₀ ratio (IC₅₀ Isotype/ IC₅₀ Targeting)** | | | |
|---|---|---|---|---|
| | **TPP-14890-TAC CAR 0.7** | **TPP-29537-TAC CAR 0.5** | **TPP-14890-TAC CAR 10** | **TPP-29537-TAC CAR 11** |
| **HepG2** | 159 | 252 | 66 | 151 |
| **Hep3B2.1-7** | 17 | 27 | 4 | 19 |

Strong and specific cytotoxicity was observed in HepG2 and Hep3B2.1-7 cell lines. Highest specific cytotoxicity was determined for TPP-29537-TAC CAR 0.5 shown with highest IC₅₀ ratio. The high CAR TPP-29537 conjugate demonstrated similar IC₅₀ as the low CAR TPP-14890-TAC. TPP-14890-TAC CAR 10 showed significantly lower specific cytotoxicity especially in the Hep3B2.1-7 cell line, indicating less efficient cytotoxicity compared to the other tested compounds, indicating an impaired binding of high CAR TPP-14890-TAC due to labeling of one or more of the overall four lysine residues within the CDRs of prior art antibody TPP-14890.

### Sequences

**Table 14**

| **TPP ID** | **Seq. Name** | **Seq. Regio n** | **Seq Type** | **SEQ ID NO** | **SEQ** |
|---|---|---|---|---|---|
| TPP-14890 | Codrituz umab-hIgG1K appa | VH | PRT | 1 | QVQLVQSGAEVKKPGASVKVSCKASGYTFTDYEMHWV RQAPGQGLEWMGALDPKTGDTAYSQKFKGRVTLTADK STSTAYMELSSLTSEDTAVYYCTRFYSYTYWGQGTLVT VSS |
| TPP-14890 | Codrituz umab-hIgG1K appa | HCDR 1 | PRT | 2 | DYEMH |
| TPP-14890 | Codrituz umab-hIgG1K appa | HCDR 2 | PRT | 3 | ALDPKTGDTAYSQKFKG |
| TPP-14890 | Codrituz umab-hIgG1K appa | HCDR 3 | PRT | 4 | FYSYTY |
| TPP-14890 | Codrituz umab-hIgG1K appa | VL | PRT | 5 | DVVMTQSPLSLPVTPGEPASISCRSSQSLVHSNRNTYL HWYLQKPGQSPQLLIYKVSNRFSGVPDRFSGSGSGTD FTLKISRVEAEDVGVYYCSQNTHVPPTFGQGTKLEIK |
| TPP-14890 | Codrituz umab-hIgG1K appa | LCDR 1 | PRT | 6 | RSSQSLVHSNRNTYLH |
| TPP-14890 | Codrituz umab-hIgG1K appa | LCDR 2 | PRT | 7 | KVSNRFS |
| TPP-14890 | Codrituz umab-hIgG1K appa | LCDR 3 | PRT | 8 | SQNTHVPPT |
| TPP-14890 | Codrituz umab-hIgG1K appa | Heavy Chain | PRT | 9 | QVQLVQSGAEVKKPGASVKVSCKASGYTFTDYEMHWV RQAPGQGLEWMGALDPKTGDTAYSQKFKGRVTLTADK STSTAYMELSSLTSEDTAVYYCTRFYSYTYWGQGTLVT VSSASTKGPSVFPLAPSSKSTSGGTAALGCLVKDYFPE PVTVSWNSGALTSGVHTFPAVLQSSGLYSLSSVVTVPS SSLGTQTYICNVNHKPSNTKVDKKVEPKSCDKTHTCPP CPAPELLGGPSVFLFPPKPKDTLMISRTPEVTCVVVDVS HEDPEVKFNWYVDGVEVHNAKTKPREEQYNSTYRVVS VLTVLHQDWLNGKEYKCKVSNKALPAPIEKTISKAKGQP REPQVYTLPPSRDELTKNQVSLTCLVKGFYPSDIAVEW ESNGQPENNYKTTPPVLDSDGSFFLYSKLTVDKSRWQ QGNVFSCSVMHEALHNHYTQKSLSLSPGK |
| TPP-14890 | Codrituz umab-hIgG1K appa | Light Chain | PRT | 10 | DVVMTQSPLSLPVTPGEPASISCRSSQSLVHSNRNTYL HWYLQKPGQSPQLLIYKVSNRFSGVPDRFSGSGSGTD FTLKISRVEAEDVGVYYCSQNTHVPPTFGQGTKLEIKRT VAAPSVFIFPPSDEQLKSGTASVVCLLNNFYPREAKVQ |
| | | | | | WKVDNALQSGNSQESVTEQDSKDSTYSLSSTLTLSKAD YEKHKVYACEVTHQGLSSPVTKSFNRGEC |
| TPP-29537 | 14890-gl53-gl65_hlg G1Kapp a | VH | PRT | 11 | QVQLVQSGAEVKKPGASVKVSCKASGYTFTDYEMHWV RQAPGQGLEWMGALDPSTGSTSYAQRFQGRVTMTRD TSTSTVYMELSSLRSEDTAVYYCTRFYSYTYWGQGTLV TVSS |
| TPP-29537 | 14890-gl53-gl65_hlg G1Kapp a | HCDR 1 | PRT | 12 | DYEMH |
| TPP-29537 | 14890-gl53-gl65_hlg G1Kapp a | HCDR 2 | PRT | 13 | ALDPSTGSTSYAQRFQG |
| TPP-29537 | 14890-gl53-gl65_hlg G1Kapp a | HCDR 3 | PRT | 14 | FYSYTY |
| TPP-29537 | 14890-gl53-gl65_hlg G1Kapp a | VL | PRT | 15 | DIVMTQSPLSLPVTPGEPASISCRSSQSLLHSNGYNYLH WYLQKPGQSPQLLIYRGSNRASGVPDRFSGSGSGTDF TLKISRVEAEDVGVYYCSQNTHVPYTFGQGTKLEIK |
| TPP-29537 | 14890-gl53-gl65_hlg G1Kapp a | LCDR 1 | PRT | 16 | RSSQSLLHSNGYNYLH |
| TPP-29537 | 14890-gl53-gl65_hlg G1Kapp a | LCDR 2 | PRT | 17 | RGSNRAS |
| TPP-29537 | 14890-gl53-gl65_hlg G1Kapp a | LCDR 3 | PRT | 18 | SQNTHVPYT |
| TPP-29537 | 14890-gl53-gl65_hlg G1Kapp a | VH | DNA | 19 | CAGGTTCAGCTGGTTCAGTCTGGCGCCGAAGTGAAG AAACCTGGCGCCTCTGTGAAGGTGTCCTGCAAGGCC AGCGGCTACACCTTTACCGACTACGAGATGCACTGG GTCCGACAGGCTCCAGGACAAGGCTTGGAATGGATG GGAGCCCTGGATCCTAGCACCGGCAGCACATCTTAC GCCCAGAGATTCCAGGGCAGAGTGACCATGACCAGA GACACCAGCACCTCCACCGTGTACATGGAACTGAGC AGCCTGAGAAGCGAGGACACCGCCGTGTACTACTGC ACCCGGTTCTACAGCTACACCTACTGGGGCCAGGGC ACCCTGGTCACAGTTTCTTCA |
| TPP-29537 | 14890-gl53-gl65_hlg G1Kapp a | HCDR 1 | DNA | 20 | GACTACGAGATGCAC |
| TPP-29537 | 14890-gl53-gl65_hlg G1Kapp a | HCDR 2 | DNA | 21 | GCCCTGGATCCTAGCACCGGCAGCACATCTTACGCC CAGAGATTCCAGGGC |
| TPP-29537 | 14890-gl53-gl65_hlg G1Kapp a | HCDR 3 | DNA | 22 | TTCTACAGCTACACCTAC |
| TPP-29537 | 14890-gl53-gl65_hlg G1Kapp a | VL | DNA | 23 | GACATCGTGATGACACAGAGCCCTCTGAGCCTGCCT GTGACACCTGGCGAACCTGCCAGCATCAGCTGTAGA AGCAGCCAGTCTCTGCTGCACAGCAACGGCTACAAC TACCTGCACTGGTATCTGCAGAAGCCCGGCCAGTCT CCTCAGCTGCTGATCTACAGAGGCAGCAACAGAGCC AGCGGCGTGCCCGATAGATTTTCTGGCTCTGGCAGC GGCACCGACTTCACCCTGAAGATCTCTAGAGTGGAA GCCGAGGACGTGGGCGTGTACTACTGCAGCCAGAAT ACCCACGTGCCATACACCTTTGGCCAGGGCACCAAG CTGGAAATCAAA |
| TPP-29537 | 14890-gl53-gl65_hlg G1Kapp a | LCDR 1 | DNA | 24 | AGAAGCAGCCAGTCTCTGCTGCACAGCAACGGCTAC AACTACCTGCAC |
| TPP-29537 | 14890-gl53-gl65_hlg G1Kapp a | LCDR 2 | DNA | 25 | AGAGGCAGCAACAGAGCCAGC |
| TPP-29537 | 14890-gl53-gl65_hlg G1Kapp a | LCDR 3 | DNA | 26 | AGCCAGAATACCCACGTGCCATACACC |
| TPP-29537 | 14890-gl53-gl65_hlg G1Kapp a | Heavy Chain | PRT | 27 | QVQLVQSGAEVKKPGASVKVSCKASGYTFTDYEMHWV RQAPGQGLEWMGALDPSTGSTSYAQRFQGRVTMTRD TSTSTVYMELSSLRSEDTAVYYCTRFYSYTYWGQGTLV TVSSASTKGPSVFPLAPSSKSTSGGTAALGCLVKDYFP EPVTVSWNSGALTSGVHTFPAVLQSSGLYSLSSVVTVP SSSLGTQTYICNVNHKPSNTKVDKKVEPKSCDKTHTCP PCPAPELLGGPSVFLFPPKPKDTLMISRTPEVTCVVVDV SHEDPEVKFNWYVDGVEVHNAKTKPREEQYNSTYRVV SVLTVLHQDWLNGKEYKCKVSNKALPAPIEKTISKAKGQ PREPQVYTLPPSRDELTKNQVSLTCLVKGFYPSDIAVE WESNGQPENNYKTTPPVLDSDGSFFLYSKLTVDKSRW QQGNVFSCSVMHEALHNHYTQKSLSLSPGK |
| TPP-29537 | 14890-gl53-gl65_hlg G1Kapp a | Light Chain | PRT | 28 | DIVMTQSPLSLPVTPGEPASISCRSSQSLLHSNGYNYLH WYLQKPGQSPQLLIYRGSNRASGVPDRFSGSGSGTDF TLKISRVEAEDVGVYYCSQNTHVPYTFGQGTKLEIKRTV AAPSVFIFPPSDEQLKSGTASVVCLLNNFYPREAKVQW KVDNALQSGNSQESVTEQDSKDSTYSLSSTLTLSKADY EKHKVYACEVTHQGLSSPVTKSFNRGEC |
| TPP-29537 | 14890-gl53-gl65_hlg | Heavy Chain | DNA | 29 | CAGGTTCAGCTGGTTCAGTCTGGCGCCGAAGTGAAG AAACCTGGCGCCTCTGTGAAGGTGTCCTGCAAGGCC AGCGGCTACACCTTTACCGACTACGAGATGCACTGG GTCCGACAGGCTCCAGGACAAGGCTTGGAATGGATG |
| | G1Kapp a | | | | GGAGCCCTGGATCCTAGCACCGGCAGCACATCTTAC GCCCAGAGATTCCAGGGCAGAGTGACCATGACCAGA GACACCAGCACCTCCACCGTGTACATGGAACTGAGC AGCCTGAGAAGCGAGGACACCGCCGTGTACTACTGC ACCCGGTTCTACAGCTACACCTACTGGGGCCAGGGC ACCCTGGTCACAGTTTCTTCAGCCAGCACCAAGGGC CCCAGCGTGTTCCCTCTGGCCCCTAGCAGCAAGAGC ACATCTGGCGGAACAGCCGCCCTGGGCTGCCTCGTG AAGGACTACTTTCCCGAGCCCGTGACCGTGTCCTGG AACTCTGGCGCTCTGACAAGCGGCGTGCACACCTTT CCAGCCGTGCTGCAGAGCAGCGGCCTGTACTCTCTG AGCAGCGTCGTGACAGTGCCCAGCAGCTCTCTGGGC ACCCAGACCTACATCTGCAACGTGAACCACAAGCCCA GCAACACCAAGGTGGACAAGAAGGTGGAACCCAAGA GCTGCGACAAGACCCACACCTGTCCCCCTTGTCCTG CCCCCGAACTGCTGGGAGGCCCTTCCGTGTTCCTGT TCCCCCCAAAGCCCAAGGACACCCTGATGATCAGCC GGACCCCCGAAGTGACCTGCGTGGTGGTGGATGTGT CCCACGAGGACCCTGAAGTGAAGTTCAATTGGTACGT GGACGGCGTGGAAGTGCACAACGCCAAGACCAAGCC TAGAGAGGAACAGTACAACAGCACCTACCGGGTGGT GTCCGTGCTGACAGTGCTGCACCAGGACTGGCTGAA CGGCAAAGAGTACAAGTGCAAGGTGTCCAACAAGGC CCTGCCTGCCCCCATCGAGAAAACCATCAGCAAGGC CAAGGGCCAGCCCCGCGAACCCCAGGTGTACACACT GCCCCCAAGCAGGGACGAGCTGACCAAGAACCAGGT GTCCCTGACCTGTCTCGTGAAAGGCTTCTACCCCTCC GATATCGCCGTGGAATGGGAGAGCAACGGCCAGCCC GAGAACAACTACAAGACCACCCCCCCTGTGCTGGAC AGCGACGGCTCATTCTTCCTGTACAGCAAGCTGACC GTGGACAAGTCCCGGTGGCAGCAGGGCAACGTGTTC AGCTGCAGCGTGATGCACGAGGCCCTGCACAACCAC TACACCCAGAAGTCCCTGAGCCTGAGCCCTGGCAAG |
| TPP-29537 | 14890-gl53-gl65_hlg G1Kapp a | Light Chain | DNA | 30 | GACATCGTGATGACACAGAGCCCTCTGAGCCTGCCT GTGACACCTGGCGAACCTGCCAGCATCAGCTGTAGA AGCAGCCAGTCTCTGCTGCACAGCAACGGCTACAAC TACCTGCACTGGTATCTGCAGAAGCCCGGCCAGTCT CCTCAGCTGCTGATCTACAGAGGCAGCAACAGAGCC AGCGGCGTGCCCGATAGATTTTCTGGCTCTGGCAGC GGCACCGACTTCACCCTGAAGATCTCTAGAGTGGAA GCCGAGGACGTGGGCGTGTACTACTGCAGCCAGAAT ACCCACGTGCCATACACCTTTGGCCAGGGCACCAAG CTGGAAATCAAACGAACCGTGGCCGCTCCCAGCGTG TTCATCTTCCCACCTAGCGACGAGCAGCTGAAGTCCG GCACAGCCTCTGTCGTGTGCCTGCTGAACAACTTCTA CCCCCGCGAGGCCAAGGTGCAGTGGAAGGTGGACA ATGCCCTGCAGAGCGGCAACAGCCAGGAAAGCGTGA CCGAGCAGGACAGCAAGGACTCCACCTACAGCCTGA GCAGCACCCTGACCCTGAGCAAGGCCGACTACGAGA AGCACAAGGTGTACGCCTGCGAAGTGACCCACCAGG GCCTGTCTAGCCCCGTGACCAAGAGCTTCAACCGGG GCGAGTGT |
| TPP-32711 | 32695 - Nanobo dy-VHH-myc-his | VHH (nanob ody) | PRT | 31 | QVQLVQSGGGLVQPGGSLRLSCAASYFDFDSYEMSWV RQAPGKGLEWIGSIYHSGSTYYNPSLKSRVTISRDNSKN TLYLQMNTLRAEDTATYYCARVNMDRFDYWGQGTLVT VSSSAGEQKLISEEDLHHHHHH |
| APP-10500 | 14890-gl53-gl65_hlg G1Kapp a_clv(hF ab)_TP P-29537-b | Fab Heavy Chain | PRT | 32 | QVQLVQSGAEVKKPGASVKVSCKASGYTFTDYEMHWV RQAPGQGLEWMGALDPSTGSTSYAQRFQGRVTMTRD TSTSTVYMELSSLRSEDTAVYYCTRFYSYTYWGQGTLV TVSSASTKGPSVFPLAPSSKSTSGGTAALGCLVKDYFP EPVTVSWNSGALTSGVHTFPAVLQSSGLYSLSSVVTVP SSSLGTQTYICNVNHKPSNTKVDKKVEPKSCDKTH |
| APP-10500 | 14890-gl53-gl65_hlg G1Kapp a_clv(hF ab)_TP P-29537-b | Fab Light Chain | PRT | 33 | DIVMTQSPLSLPVTPGEPASISCRSSQSLLHSNGYNYLH WYLQKPGQSPQLLIYRGSNRASGVPDRFSGSGSGTDF TLKISRVEAEDVGVYYCSQNTHVPYTFGQGTKLEIKRTV AAPSVFIFPPSDEQLKSGTASVVCLLNNFYPREAKVQW KVDNALQSGNSQESVTEQDSKDSTYSLSSTLTLSKADY EKHKVYACEVTHQGLSSPVTKSFNRGEC |
| APP-277 | M014-G07 Fab-TPP-754 | Fab Heavy Chain | PRT | 34 | EVQLLESGGGLVQPGGSLRLSCAASGFTFGDYAMSWV RQAPGKGLEWVSGISGSGGSTYYADSVKGRFTISRDNS KNTLYLQMNSLRAEDTAVYYCAREGETSFGLDVWGQG TLVTVSSASTKGPSVFPLAPSSKSTSGGTAALGCLVKDY FPEPVTVSWNSGALTSGVHTFPAVLQSSGLYSLSSVVT VPSSSLGTQTYICNVNHKPSNTKVDKKVEPKSCDKTH |
| APP-277 | M014-G07 Fab-TPP-754 | Fab Light Chain | PRT | 35 | QSVLTQPPSASGTPGQRVTISCSGSSSNIGSNYVYWYQ QLPGTAPKLLIYDNNDRPSGVPDRFSGSKSGTSASLAIS GLRSEDEADYYCVAWDDSLNGHWVFGGGTKLTVLGQP KAAPSVTLFPPSSEELQANKATLVCLISDFYPGAVTVAW KADSSPVKAGVETTTPSKQSNNKYAASSYLSLTPEQWK SHRSYSCQVTHEGSTVEKTVAPTECS |
| APP-10499 | 14890-gl53-gl65_hlg G1Kapp a_clv(hF ab)_TP P-29537-a | Fab Heavy Chain + dimeri sation part | PRT | 36 | QVQLVQSGAEVKKPGASVKVSCKASGYTFTDYEMHWV RQAPGQGLEWMGALDPSTGSTSYAQRFQGRVTMTRD TSTSTVYMELSSLRSEDTAVYYCTRFYSYTYWGQGTLV TVSSASTKGPSVFPLAPSSKSTSGGTAALGCLVKDYFP EPVTVSWNSGALTSGVHTFPAVLQSSGLYSLSSVVTVP SSSLGTQTYICNVNHKPSNTKVDKKVEPKSCDKTHTCP PCPAPELLG |
| APP-10499 | 14890-gl53-gl65_hlg G1Kapp a_clv(hF ab)_TP P-29537-a | Fab Light Chain Presen t twice | PRT | 37 | IVMTQSPLSLPVTPGEPASISCRSSQSLLHSNGYNYLHW YLQKPGQSPQLLIYRGSNRASGVPDRFSGSGSGTDFTL KISRVEAEDVGVYYCSQNTHVPYTFGQGTKLEIKRTVAA PSVFIFPPSDEQLKSGTASVVCLLNNFYPREAKVQWKV DNALQSGNSQESVTEQDSKDSTYSLSSTLTLSKADYEK HKVYACEVTHQGLSSPVTKSFNRGEC |
| APP-6320 | (M014-G07-hIgG1-lambda_ clv_TPP -754-a) | Fab Heavy Chain Presen t twice | PRT | 38 | EVQLLESGGGLVQPGGSLRLSCAASGFTFGDYAMSWV RQAPGKGLEWVSGISGSGGSTYYADSVKGRFTISRDNS KNTLYLQMNSLRAEDTAVYYCAREGETSFGLDVWGQG TLVTVSSASTKGPSVFPLAPSSKSTSGGTAALGCLVKDY FPEPVTVSWNSGALTSGVHTFPAVLQSSGLYSLSSVVT VPSSSLGTQTYICNVNHKPSNTKVDKKVEPKSCDKTHT CPPCPAPELLG |
| APP-6320 | (M014-G07-hIgG1-lambda_ clv_TPP -754-a) | Fab Light Chain Presen t twice | PRT | 39 | QSVLTQPPSASGTPGQRVTISCSGSSSNIGSNYVYWYQ QLPGTAPKLLIYDNNDRPSGVPDRFSGSKSGTSASLAIS GLRSEDEADYYCVAWDDSLNGHWVFGGGTKLTVLGQP KAAPSVTLFPPSSEELQANKATLVCLISDFYPGAVTVAW KADSSPVKAGVETTTPSKQSNNKYAASSYLSLTPEQWK SHRSYSCQVTHEGSTVEKTVAPTECS |
| APP-10652 | 14890-scFv-Fc-hIgG1_c Iv_TPP-30783-a | scFv2 one chain | PRT | 40 | QVQLVQSGAEVKKPGASVKVSCKASGYTFTDYEMHWV RQAPGQGLEWMGALDPKTGDTAYSQKFKGRVTLTADK STSTAYMELSSLTSEDTAVYYCTRFYSYTYWGQGTLVT VSSGGGGSGGGGSGGGGSDVVMTQSPLSLPVTPGEP ASISCRSSQSLVHSNRNTYLHWYLQKPGQSPQLLIYKVS NRFSGVPDRFSGSGSGTDFTLKISRVEAEDVGVYYCSQ NTHVPPTFGQGTKLEIKGGGGSGGGGSGGGGSGVEC PPCPAPELLG |
| TPP-30783 | 14890-scFv-Fc-hIgG1 PRT seq. is present twice | ScFv-Fc | PRT | 41 | QVQLVQSGAEVKKPGASVKVSCKASGYTFTDYEMHWV RQAPGQGLEWMGALDPKTGDTAYSQKFKGRVTLTADK STSTAYMELSSLTSEDTAVYYCTRFYSYTYWGQGTLVT VSSGGGGSGGGGSGGGGSDVVMTQSPLSLPVTPGEP ASISCRSSQSLVHSNRNTYLHWYLQKPGQSPQLLIYKVS NRFSGVPDRFSGSGSGTDFTLKISRVEAEDVGVYYCSQ NTHVPPTFGQGTKLEIKGGGGSGGGGSGGGGSGVEC PPCPAPELLGGPSVFLFPPKPKDTLMISRTPEVTCVVVD VSHEDPEVKFNWYVDGVEVHNAKTKPREEQYNSTYRV VSVLTVLHQDWLNGKEYKCKVSNKALPAPIEKTISKAKG QPREPQVYTLPPSRDELTKNQVSLTCLVKGFYPSDIAVE WESNGQPENNYKTTPPVLDSDGSFFLYSKLTVDKSRW QQGNVFSCSVMHEALHNHYTQKSLSLSPG |
| | Human GPC3 | | PRT | 42 | QPPPPPPDATCHQVRSFFQRLQPGLKWVPETPVPGSD LQVCLPKGPTCCSRKMEEKYQLTARLNMEQLLQSASM ELKFLIIQNAAVFQEAFEIVVRHAKNYTNAMFKNNYPSLT PQAFEFVGEFFTDVSLYILGSDINVDDMVNELFDSLFPVI YTQLMNPGLPDSALDINECLRGARRDLKVFGNFPKLIMT QVSKSLQVTRIFLQALNLGIEVINTTDHLKFSKDCGRMLT RMWYCSYCQGLMMVKPCGGYCNVVMQGCMAGVVEID KYWREYILSLEELVNGMYRIYDMENVLLGLFSTIHDSIQY VQKNAGKLTTTIGKLCAHSQQRQYRSAYYPEDLFIDKKV LKVAHVEHEETLSSRRRELIQKLKSFISFYSALPGYICSH SPVAENDTLCWNGQELVERYSQKAARNGMKNQFNLHE LKMKGPEPVVSQIIDKLKHINQLLRTMSMPKGRVLDKNL DEEGFESGDCGDDEDECIGGSGDGMIKVKNQLRFLAEL AYDLDVDDAPGNSQQATPKDNEISTFHNLGNVHSPLKL LTSMAISVVCFFFLVH |

## Claims

1. Imaging agent comprising a targeting moiety capable of binding GPC3, a chelator and a radionuclide.

2. Imaging agent according to claim 1 wherein the radionuclide is selected from the group consisting of ¹⁸ F, ⁴³Sc, ⁴⁴Sc, ⁴⁷Sc, ⁶⁷Cu, ⁶⁷Ga, ⁷¹Ge, ⁷⁷Br, ⁸⁹Sr, ⁸⁹Zr, ⁹⁰Y, ^{99m}Tc, ¹⁰³Pd, ¹⁰⁵Rh,¹¹¹In, ¹²³I, ¹²⁵I, ²²⁷Th, ¹³¹I, ¹⁴⁰Nd, ¹⁴⁹Tb, ¹⁴⁹Pm,¹⁵²Tb, ¹⁵⁵Tb, ¹⁶¹Tb, ¹⁶⁶Ho, ¹⁷⁷Lu, ¹⁷⁸_{Ta,} ¹⁸⁶Re, ¹⁸⁸Re, ¹⁹³Pt, ^{195m}Pt, ¹⁹⁷Hg, ¹⁹⁸Au, ²¹¹At, ²¹²Bi, ²¹²Pb, ²¹³Bi, ²²³Ra, ²²⁴Ra, ²²⁵Ac and ²³²Th.

3. Imaging agent according to claim 1 or claim 2 wherein the radionuclide is detectable by positron emission tomography (PET).

4. Imaging agent according to claim 1 comprising either ⁸⁹Zr and DFO* or ¹⁸F and RESCA as radionuclide and chelator, respectively.

5. Imaging agent according to claim 1 wherein the targeting moiety capable of binding GPC3 is a Fab antigen-binding fragment comprising a heavy chain antigen-binding region that comprises an H-CDR1 SEQ ID NO: 12, H-CDR2 SEQ ID NO: 13, H-CDR3 SEQ ID NO: 14 and a light chain antigen-binding region that comprises an L-CDR1 SEQ ID NO: 16, L-CDR2 SEQ ID NO: 17, L-CDR3 SEQ ID NO:18.

6. Imaging agent according to claim 1 wherein the targeting moiety capable of binding GPC3 is a Fab antigen-binding fragment comprising a heavy chain antigen-binding region that comprises an H-CDR1 SEQ ID NO: 12, H-CDR2 SEQ ID NO: 13, H-CDR3 SEQ ID NO: 14 and a light chain antigen-binding region that comprises an L-CDR1 SEQ ID NO: 16, L-CDR2 SEQ ID NO: 17, L-CDR3 SEQ ID NO:18 the chelator is RESCA and the radionuclide is ¹⁸F or the chelator is DFO* and the radionuclide is ⁸⁹Zr.

7. Imaging agent according to claim 1 wherein the targeting moiety capable of binding GPC3 is the Fab antigen-binding fragment consisting of the heavy chain of SEQ ID NO: 32 and the light chain of SEQ ID NO: 33 the chelator is RESCA and the radionuclide is ¹⁸F.

8. Imaging agent according to claim 1 having the formula (I) 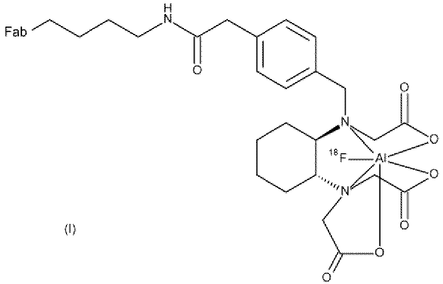 wherein Fab is the antigen-binding fragment capable of binding to GPC3 comprising a heavy chain antigen-binding region that comprises H-CDR1 SEQ ID NO: 12, H-CDR2 SEQ ID NO: 13, H-CDR3 SEQ ID NO: 14 and a light chain antigen-binding region that comprises an L-CDR1 SEQ ID NO: 16, L-CDR2 SEQ ID NO: 17, L-CDR3 SEQ ID NO:18.
